(19) Europäisches Patentamt
European Patent Office
Office européen des brevets

(11) Publication number: **0 649 834 A1**

# (12) EUROPEAN PATENT APPLICATION

(21) Application number: **93116968.4**

(22) Date of filing: **20.10.93**

(51) Int. Cl.[6]: **C07C 229/06**, C07C 229/08, C07C 229/12, A61K 7/50, A61K 7/48, A61K 7/06, C11D 1/90, C11D 1/92, C07C 309/14, C07C 275/14, C07D 207/26

(43) Date of publication of application:
**26.04.95 Bulletin 95/17**

(84) Designated Contracting States:
**DE FR GB**

(71) Applicant: **KAO CORPORATION**
**14-10, Nihonbashi,**
**Kayabacho 1-chome**
**Chuo-ku,**
**Tokyo (JP)**

(72) Inventor: **Kitsuki, Tomohito**
**1450, Nishihama**
**Wakayama-shi,**
**Wakayama (JP)**
Inventor: **Uno, Mitsuru**
**1130, Nishihama**
**Wakayama-shi,**
**Wakayama (JP)**
Inventor: **Kita, Katsumi**
**1655, Nagataki**
**Izumisano-shi,**
**Osaka (JP)**
Inventor: **Imai, Kazuyasu**
**10-10, Azusawa 2-chome**
**Itabashi-ku,**
**Tokyo (JP)**
Inventor: **Fujikura, Yoshiaki**
**271-6, Yamamoto-cho**
**Utsunomiya-shi,**
**Tochigi (JP)**
Inventor: **Nakano, Akiko**
**131, Hishiya-higashi**
**Higashi-Osaka-shi,**
**Osaka (JP)**
Inventor: **Moriyama, Masaaki**
**2090, Oaza-Hiranuma,**
**Yoshikawa-machi**
**Kita-Katsushika-gun,**
**Saitama (JP)**
Inventor: **Kajihara, Yasushi**
**Itopia 506, 6696-1 Kasukabe**
**Kasukabe-shi,**
**Saitama (JP)**
Inventor: **Arisawa, Masatoshi**
**A-208, Sun Green Matsudo**
**523-8, Koyama**
**Matsudo-shi,**
**Chiba (JP)**
Inventor: **Ataka, Yoshiharu**
**1130, Nishihama**
**Wakayama-shi,**
**Wakayama (JP)**

(74) Representative: **Hansen, Bernd, Dr. Dipl.-Chem. et al**
**Hoffmann, Eitle & Partner,**
**Patentanwälte,**
**Arabellastrasse 4**
**D-81925 München (DE)**

(54) **Carboxybetaine and sulfobetaine and detergent composition and cosmetic containing the same.**

(57) Novel carboxybetaines and sulfobetaines are disclosed. Processes of the production of the carboxybetaines and sulfobetaines, as well as detergent compositions and cosmetics containing these compounds are also disclosed. The carboxybetaines and sulfobetaines of the present invention show excellent moisture keeping effect which provides the skin and hair with moist feeling for a prolonged period of time.



Rank Xerox (UK) Business Services
(3.10/3.09/3.3.4)

## FIELD OF THE INVENTION

This invention relates to a novel carboxybetaine and sulfobetaine, their production processes, and a detergent composition and cosmetic using the carboxybetaine or the sulfobetaine. More particularly, it relates to a novel carboxybetaine and sulfobetaine which is useful as a base material and moisture keeping agent of hair cosmetics, skin cosmetics and the like, to production processes thereof, and a detergent composition and cosmetic using the carboxybetaine or sulfobetaine.

## BACKGROUND OF THE INVENTION

In general, in order to provide hair and the skin with a moist feeling, shampoos, rinses, cosmetics and the like are frequently blended with various types of moisture keeping agents such as for example glycerol, propylene glycol, sorbitol, urea and alkylene oxide addition products of saccharides.

However, these prior art moisture keeping agents are not always satisfactory in terms of their moisture keeping ability, sense of touch and the like, and their effects cannot be sustained because they are apt to diffuse and removed by sweat, water and the like. In addition, in the case of wash-off type cosmetics such as a rinse, a body rinse and the like, as well as a detergent which contains a surface active agent in a large quantity, the moisture keeping agent contained therein frequently fails to exert its intrinsic effects because the greater part of the agent is washed away when used.

In view of the above, great concern has been directed toward the development of a compound which has excellent moisture keeping ability, sense of touch and the like and can maintain its moisture keeping effect for a prolonged period of time even after its exposure to sweat and water or rinsing.

## SUMMARY OF THE INVENTION

Taking the aforementioned circumstances into consideration, the present inventors have conducted intensive studies and, as a result, found that carboxybetaines and sulfobetaines specified by the following formulae have an excellent moisture keeping ability, can be produced at a low cost and are useful as a base material and moisture keeping agent for various detergent compositions, hair and skin cosmetics and the like. The present invention has been accomplished on the basis of this finding.

Namely, according to the present invention, there is provided a carboxybetaine represented by the following formula (1):

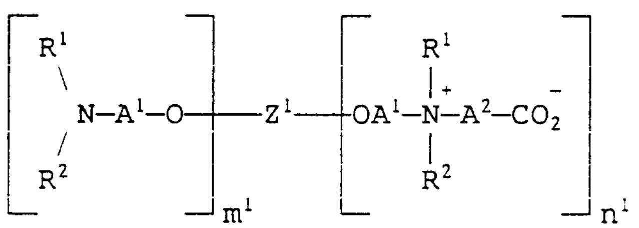

(1)

wherein $Z^1$ represents a residue remaining after removal of $m^1 + n^1$ hydroxyl groups from glycerol or a glycerol condensate; $R^1$ and $R^2$ are the same or different and each represents hydrogen atom or methyl group; $A^1$ and $A^2$ are the same or different and each represents a straight- or branched-chain alkylene group having 1 to 6 carbon atoms which may contain a hydroxyl group; and $m^1$ represents an integer of 0 or more and $n^1$ represents an integer of 1 or more, provided that $m^1 + n^1$ is equivalent to the valency of $Z^1$; as well as a process for the production of the carboxybetaine and a detergent composition and cosmetic containing the same.

According to the present invention, there is further provided a carboxybetaine represented by the following formula (6):

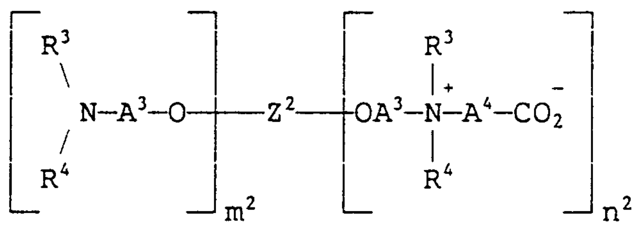

(6)

wherein $Z^2$ represents a residue remaining after removal of $m^2 + n^2$ hydroxyl groups from glycerol or a condensate thereof; $A^3$ and $A^4$ are the same or different and each represents a straight- or branched-chain alkylene group having 1 to 6 carbon atoms which may contain a hydroxyl group; $R^3$ represents a straight- or branched-chain alkyl or alkenyl group having 1 to 24 carbon atoms which may contain a hydroxyl group; $R^4$ represents a straight- or branched-chain alkyl or alkenyl group having 2 to 24 carbon atoms which may contain a hydroxyl group; and $m^2$ represents an integer of 0 or more and $n^2$ represents an integer of 1 or more, provided that $m^2 + n^2$ is equivalent to the valency of $Z^2$; as well as a process for the production of the carboxybetaine and a detergent composition and a cosmetic containing the same.

According to the present invention, there is still provided a carboxybetaine represented by the following formula (11):

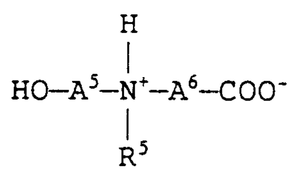

(11)

wherein $A^5$ represents a straight- or branched-chain alkylene group having 2 to 36 carbon atoms; $A^6$ represents a straight- or branched-chain alkylene group having 2 to 36 carbon atoms which may contain a hydroxyl group; and $R^5$ represents hydrogen atom or a straight- or branched-chain alkyl group having 1 to 36 carbon atoms; as well as a process for the production of the carboxybetain and a detergent composition and cosmetic containing the same.

According to the present invention, there is also provided a carboxybetaine represented by the following formula (14):

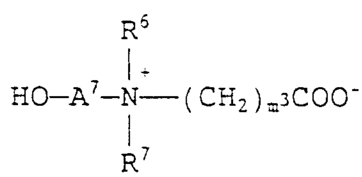

(14)

wherein $A^7$ represents a straight- or branched-chain alkylene group having 3 to 36 carbon atoms; $R^6$ and $R^7$ are the same or different and each represents hydrogen atom, a straight- or branched-chain alkyl group having 1 to 36 carbon atoms or an alkenyl group having 2 to 36 carbon atoms; and $m^3$ represents an integer of 1 to 2; as well as a process for the production of the carboxybetaine and a detergent composition and cosmetic containing the same.

According to the present invention, there is furthermore provided a carboxybetaine represented by the following formula (19):

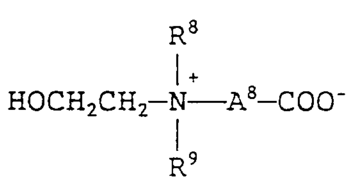

(19)

wherein $A^8$ represents a straight- or branched-chain alkylene group having 3 to 36 carbon atoms which may be substituted with a hydroxyl group; and $R^8$ and $R^9$ are the same or different and each represents a straight- or branched-chain alkyl group having 1 to 36 carbon atoms or an alkenyl group having 2 to 36 carbon atoms, as well as a process for the production of the carboxybetain and a detergent composition and cosmetic containing the same.

According to the present invention, there is yet provided a carboxybetaine represented by the following formula (22):

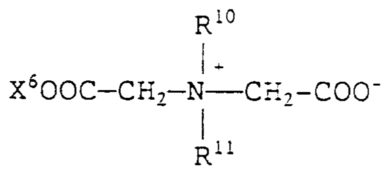

(22)

wherein $R^{10}$ and $R^{11}$ are the same or different and each represents a straight- or branched-chain alkyl group having 1 to 5 carbon atoms which may contain a hydroxy group, provided that at least one of the alkyl groups represented by $R^{10}$ and $R^{11}$ contains a hydroxy group; and $X^6$ represents hydrogen atom or a cation; as well as a process for the production of the carboxybetaine and a detergent composition and cosmetic containing the same.

According to the present invention, there is still further provided a carboxybetaine represented by the following formula (25):

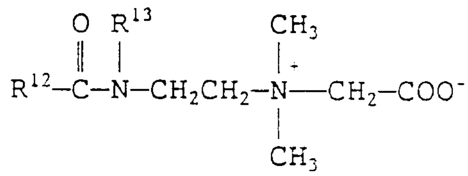

(25)

wherein $R^{12}$ represents an alkyl group having 1 to 6 carbon atoms which may be substituted with a hydroxyl group,

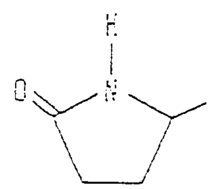

or

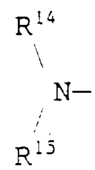

wherein $R^{14}$ and $R^{15}$ are the same or different and each represents hydrogen atom or an alkyl group having 1 to 6 carbon atoms which may be substituted with a hydroxyl group; and $R^{13}$ represents hydrogen atom or an alkyl group having 1 to 6 carbon atoms which may be substituted with a hydroxyl group; as well as a process for the production of the carboxybetaine and a detergent composition and cosmetic containing the same.

According to the present invention, there is still furthermore provided a carboxybetaine represented by the following formula (32):

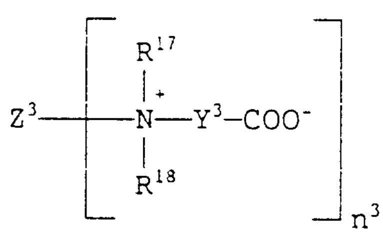

(32)

wherein $Z^3$ represents a residue remaining after removal of $n^3$ hydroxyl groups from glycerol or a condensate thereof or a group represented by HO—$A^9$— where $A^9$ represents a straight- or branched-chain alkylene group having 2 to 5 carbon atoms; $R^{17}$ and $R^{18}$ are the same or different and each represents a straight- or branched-chain alkyl group having 1 to 5 carbon atoms; $Y^3$ represents a straight- or branched-chain alkylene group which may contain a hydroxyl group; and $n^3$ is an integer of at least 1 but not exceeding the number of hydroxyl groups in glycerol or a condensate thereof and is 1 when $Z^3$ is HO—$A^9$—; as well as cosmetics and detergent compositions which contain the carboxybetaine; as well as a process for the production of the carboxybetaine and a detergent composition and cosmetic containing the same.

According to the present invention, there is yet furthermore provided a sulfobetaine represented by the following formula (35):

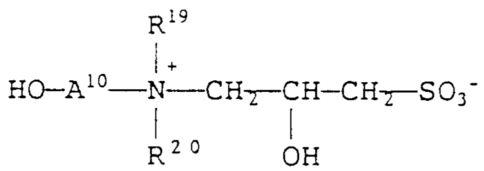

(35)

wherein $A^{10}$ represents a straight- or branched-chain alkylene group having 2 to 12 carbon atoms which may be substituted with a hydroxyl group; $R^{19}$ and $R^{20}$ are the same or different and each represents a straight- or branched-chain alkyl group having 1 to 12 carbon atoms or a straight- or branched-chain alkenyl group having 2 to 12 carbon atoms each of which may be substituted with a hydroxyl group; as well as a process for the production of the sulfobetaine and a detergent composition and cosmetic containing the same.

According to the present invention, there is yet also provided a means for providing skin or hair with a moist feel which employs a carboxybetaine represented by the following formula (14'):

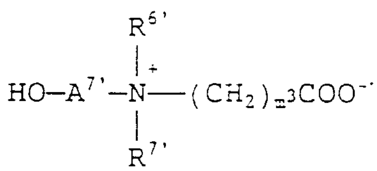

(14')

wherein $A^{7'}$ represents a straight- or branched-chain alkylene group having 2 to 36 carbon atoms; $R^{6'}$ and $R^{7'}$ are the same or different and each represents hydrogen atom, a straight- or branched-chain alkyl group having 1 to 36 carbon atoms or an alkenyl group having 2 to 36 carbon atoms each of which may be substituted with a hydroxyl group; and $m^3$ represents an integer of 1 or 2.

## BRIEF DESCRIPTION OF THE DRAWINGS

Fig. 1 is a graph showing a $^1$H-NMR spectrum of the carboxybetaine (1) of the present invention obtained in Example 1.

Fig. 2 is a graph showing a $^1$H-NMR spectrum of the carboxybetaine (1) of the present invention obtained in Example 2.

Fig. 3 is a graph showing a $^1$H-NMR chart of a compound obtained in Example 47.

Fig. 4 is a graph showing a $^1$H-NMR chart of a compound obtained in Example 48.

## DETAILED DESCRIPTION OF THE INVENTION

In the aforementioned formula (1) representing the carboxybetaine of the present invention, $Z^1$ represents a residue remaining after removal of $m^1$ + $n^1$ hydroxyl groups from glycerol or a condensate thereof. Examples of the glycerol condensate are those obtained by the condensation of glycerol into straight- or branched-chain form in the presence of an alkali catalyst, preferred examples of which include polyglycerols having an average condensation degree of 20 or less, such as diglycerol, triglycerol,

tetraglycerol, pentaglycerol, hexaglycerol, heptaglycerol, octaglycerol, nonaglycerol, decaglycerol, dodecaglycerol, tetradecaglycerol, hexadecaglycerol, octadecaglycerol and the like, of which those having an average condensation degree of 10 or less are preferred. As the group represented by $Z^1$, glycerol or diglycerol from which one hydroxyl group is removed is particularly preferred.

In formula (1), $A^1$ and $A^2$ are the same or different and each represents a straight- or branched-chain alkylene group having 1 to 6 carbon atoms. Preferred examples of $A^1$ include ethylene group, propylene group and 2-hydroxy-1,3-propylene group, and those of $A^2$ include methylene group, ethylene group, propylene group and butylene group.

Preferably, in the carboxybetaine (1), $n^1$ is 1 and $R^1$ and $R^2$ are both methyl group, or $m^1$ is 0.

The carboxybetaine (1) of the present invention can be produced for example in accordance with the following reaction formula.

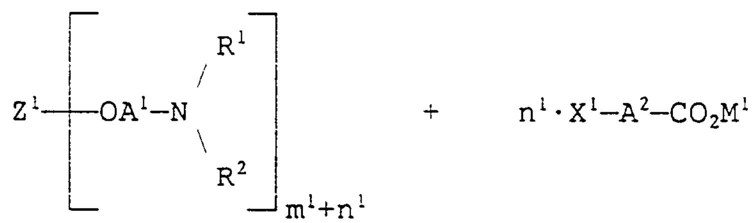

(2) (3)

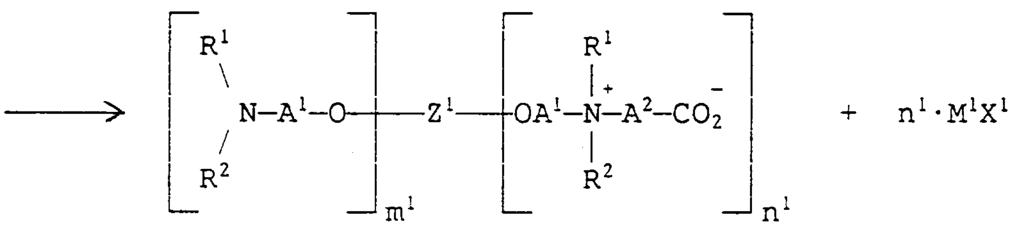

(1)

In the above formula, $Z^1$, $A^1$, $A^2$, $R^1$, $R^2$, $m^1$ and $n^1$ represent the same groups and integers as defined above, $X^1$ represents a halogen atom and $M^1$ represents a cation.

That is, the carboxybetaine (1) of the present invention can be produced by allowing an amino compound (2) to react with a compound (3).

In the practice of the above reaction, the compound (3) may be used in an amount of 1 to 5 equivalent, preferably 1 to 2 equivalent, based on the number of amino groups of the amino compound (2). The above reaction may be carried out at a temperature of from 20 to 120 °C, preferably from 40 to 90 °C in the presence of an inert solvent. Examples of the inert solvent to be used in this reaction include polar solvents such as water, methanol, ethanol, isopropyl alcohol, dimethylformamide, dimethyl sulfoxide and the like and a mixture of two or more of these solvents, of which water alone or a mixture of water and a lower alcohol is preferred. Though not particularly limited, the cation represented by $M^1$ in formula (3) may be selected from alkali metal ions, ammonium ion and alkanolammonium ions having a total carbon number of 2 to 9.

In addition to the carboxybetaine (1) of the present invention, the reaction product thus obtained contains the unreacted amino compound (2) and compound (3) and by-products. In consequence, the reaction product may be subjected if necessary to purification in conventional means such as solvent fractionation, ion exchange chromatography, electrodialysis and the like.

The compound (2) as a starting material of the present invention may be produced for example in accordance with the following known method.

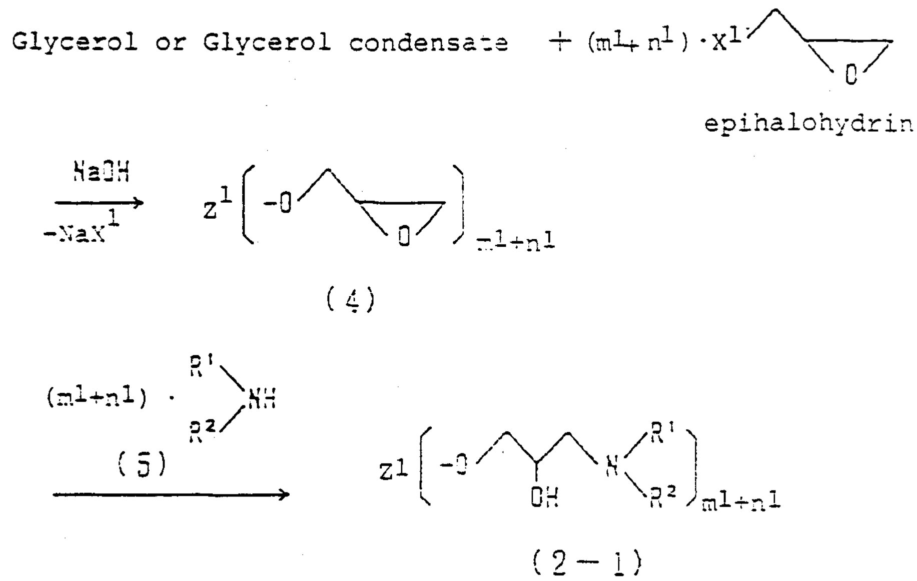


(4)

(5)

(2-1)

In the above formula, $Z^1$, $X^1$, $R^1$, $R^2$, $m^1$ and $n^1$ represent the same groups and integers as defined above.

That is, the compound (2) can be obtained by allowing glycerol or a glycerol condensate to react with an epihalohydrin and then allowing the thus formed glycidyl-etherified glycerol or glycerol condensate (4) to react with ammonia, methylamine or dimethylamine (5).

Reaction of the compound (4) with the compound (5) may be carried out at a compound (5)/compound (4) molar ratio of 1 to 2, preferably 1 to 1.5, at a reaction temperature of from room temperature to 80 °C. This reaction may be carried out in the absence of solvent or in a lower alcohol such as methanol, ethanol, isopropanol or the like or in an organic solvent such as chloroform or the like. This reaction can be effected in the absence of catalyst.

With regard to the compound (3), its illustrative examples include sodium chloroacetate, lithium bromovalerate and the like.

The following illustrates a process for the production of the carboxybetaine (1) of the present invention in which glycerol is used as a starting material.

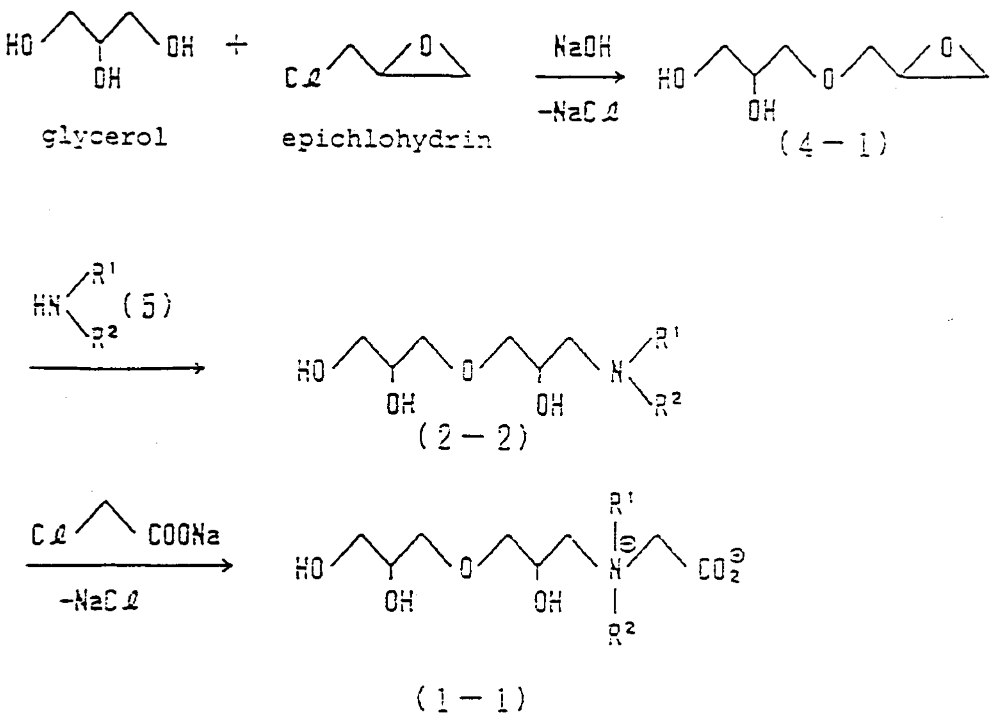


glycerol epichlohydrin (4 − 1) (2 − 2) (1 − 1)

In the above reaction formula, $R^1$ and $R^2$ represent the same groups as defined above.

In the aforementioned formula (6) representing the carboxybetaine of the present invention, $Z^2$ represents a residue remaining after removal of $m^2$ + $n^2$ hydroxyl groups from glycerol or a condensate thereof. Examples of the glycerol condensate are those obtained by the condensation of glycerol into straight- or branched-chain form in the presence of an alkali catalyst, preferred examples of which include polyglycerols having an average condensation degree of 20 or less, such as diglycerol, triglycerol, tetraglycerol, pentaglycerol, hexaglycerol, heptaglycerol, octaglycerol, nonaglycerol, decaglycerol, dodecaglycerol, tetradecaglycerol, hexadecaglycerol, octadecaglycerol and the like, of which those having an average condensation degree of 10 or less are more preferred. As the group represented by $Z^2$, glycerol or diglycerol from which one hydroxyl group is removed is particularly preferred.

In formula (6), $A^3$ and $A^4$ are the same or different and each represents a straight- or branched-chain alkylene group having 1 to 6 carbon atoms. Preferred examples of $A^3$ include ethylene group, propylene group and 2-hydroxy-1,3-propylene group, and those of $A^4$ include methylene group, ethylene group, propylene group and butylene group.

Preferably, in the carboxybetaine (6) of the present invention, $n^2$ is 1, $R^3$ is methyl group and $R^4$ is a strainght-or branched-chain alkyl group having 2 to 18 carbon atoms, or $m^2$ is 0.

In formula (6), $R^3$ represents a straight- or branched-chain alkyl or alkenyl group having 1 to 24 carbon atoms which may contain a hydroxyl group. Illustrative examples of these groups include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, ethenyl, propenyl, butenyl, pentenyl, hexenyl, heptenyl, octenyl, nonenyl, decenyl, dodecenyl, undecenyl, tridecenyl, tetradecenyl, pentadecenyl, hexadecenyl, heptadecenyl, octadecenyl, nonadecenyl, eicosenyl, heneicosenyl, docosenyl, tricosenyl, tetracosenyl, methylhexyl, ethylhexyl, methylheptyl, ethylheptyl, methylnonyl, methylundecenyl, methylheptadecanyl, hexyldecyl, octyldecyl, 2-hydroxypropyl and the like groups.

In formula (6), $R^4$ represents a straight- or branched-chain alkyl or alkenyl group having 2 to 24 carbon atoms which may contain a hydroxyl group. The groups described above as illustrative examples of $R^3$, excluding methyl group, may be used as $R^4$.

The carboxybetaine (6) of the present invention can be produced for example in accordance with the following reaction formula.

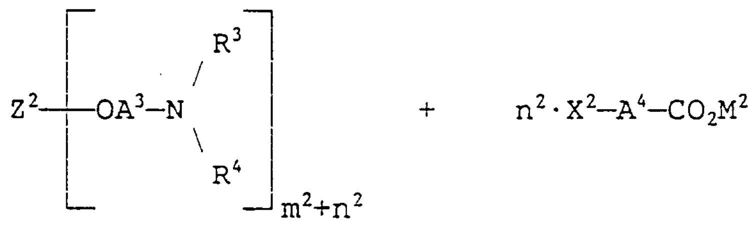

(7) (8)

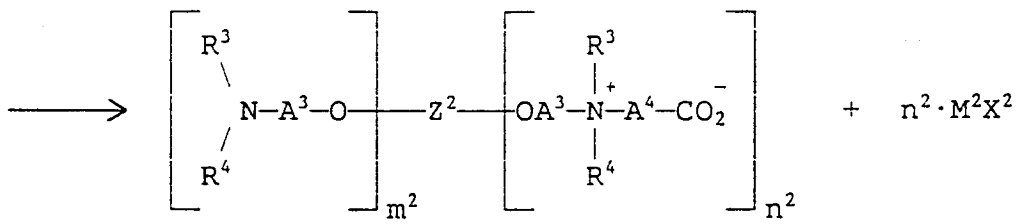

(6)

In the above formula, $Z^2$ $A^3$, $A^4$, $R^3$, $R^4$, $m^2$ and $n^2$ represent the same groups and integers as defined above, $X^2$ represents a halogen atom and $M^2$ represents a cation.

That is, the carboxybetaine (6) of the present invention can be produced by allowing an amino compound (7) to react with a compound (8).

In the practice of the above reaction, the compound (8) may be used in an amount of 1 to 5 equivalent, preferably 1 to 2 equivalent, based on the number of amino groups of the amino compound (7). The above reaction may be carried out at a temperature of from 20 to 120°C, preferably from 40 to 90°C in the presence of an inert solvent. Examples of the inert solvent to be used in this reaction include polar solvents such as water, methanol, ethanol, isopropyl alcohol, dimethylformamide, dimethyl sulfoxide and the like and a mixture of two or more of these solvents, of which water alone or a mixture of water and a lower alcohol is preferred. Though not particularly limited, the cation represented by $M^2$ in formula (8) may be selected from alkali metal ions, ammonium ion and alkanolammonium ions having a total carbon number of 2 to 9.

In addition to the carboxybetaine (6) of the present invention, the reaction product thus obtained contains unreacted amino compound (7) and compound (8) and by-products. In consequence, the reaction product may be subjected if necessary to purification in conventional means such as solvent fractionation, ion exchange chromatography, electrodialysis and the like.

The compound (7) as a starting material of the present invention may be produced for example in accordance with the following known method.

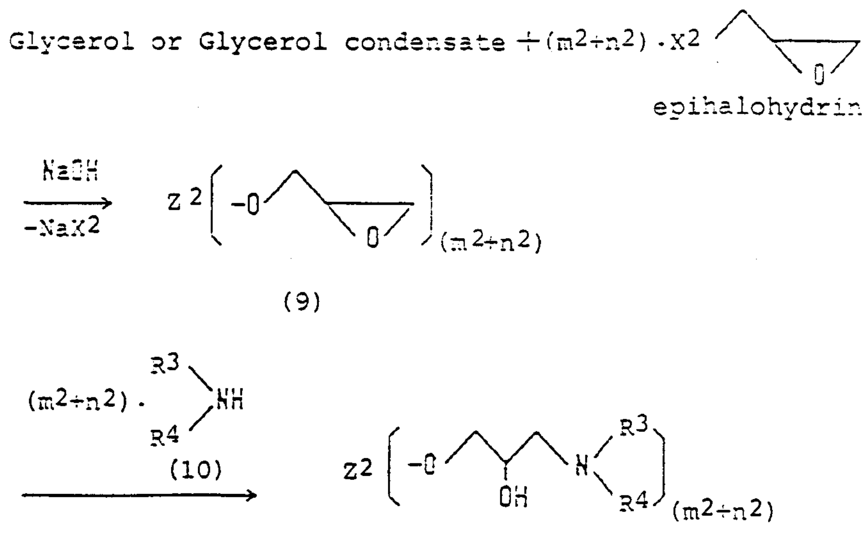


(9)

(7-1)

In the above formula, $Z^2$, $X^2$, $R^3$, $R^4$, $m^2$ and $n^2$ represent the same groups and integers as defined above.

That is, the compound (7) can be obtained by allowing glycerol or a glycerol condensate to react with an epihalohydrin and then allowing the thus formed glycidyl-etherified glycerol or glycerol condensate (9) to react with a dialkylamine (10).

Reaction of the compound (9) with the compound (10) may be carried out at a compound (10)-/compound (9) molar ratio of 1 to 2, preferably 1 to 1.5, at a reaction temperature of from room temperature to 80 °C. This reaction may be carried out in the absence of solvent or in a lower alcohol such as methanol, ethanol, isopropanol or the like or in an organic solvent such as chloroform or the like. This reaction can be effected in the absence of catalyst.

With regard to the compound (8), its illustrative examples include sodium chloroacetate, lithium bromovalerate and the like.

The following illustrates a process for the production of the carboxybetaine (6) of the present invention in which glycerol is used as a starting material.

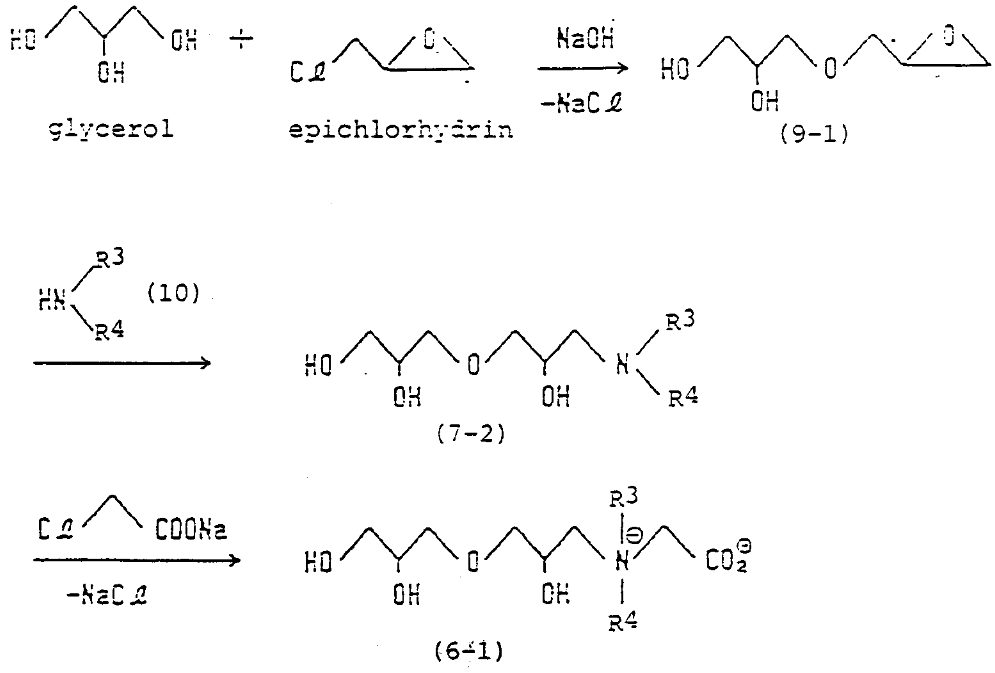


glycerol

epichlorhydrin

(9-1)

(7-2)

(6-1)

In the above reaction formula, $R^3$ and $R^4$ represent the same groups as defined above.

In the aforementioned formula (11), $A^5$ represents a straight- or branched-chain alkylene group having 2 to 36 carbon atoms. Illustrative examples of the alkylene group include ethylene, propylene, butylene, pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene and the like groups. Of these groups, those having 2 to 6 carbon atoms, more particularly ethylene group, propylene group and butylene group are preferred.

In the aforementioned formula (11), $A^6$ represents a straight- or branched-chain alkylene group having 2 to 36 carbon atoms which may contain a hydroxyl group as a substituent. Illustrative examples of the alkylene group include ethylene, propylene, butylene, pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, 2-hydroxypropylene and the like groups. Of these groups, those having 2 to 12 carbon atoms, more particularly ethylene group, propylene group, butylene group, pentylene group, hexylene group and 2-hydroxypropylene group are preferred.

In formula (11), $R^5$ represents hydrogen atom or a straight- or branched-chain alkyl group having 1 to 36 carbon atoms, with particularly preferred examples including hydrogen atom and methyl group.

The carboxybetaine (11) of the present invention can be produced for example by allowing an amine compound represented by formula (12) to react with a carboxylate represented by formula (13), in accordance with the following reaction formula.

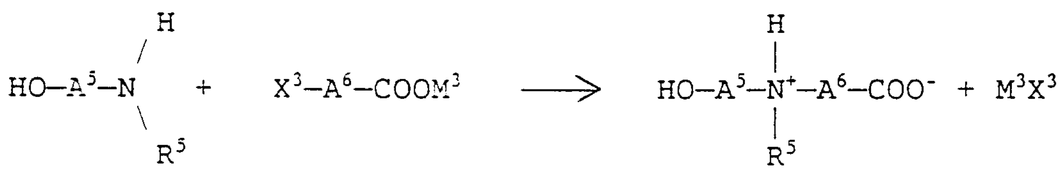

(12) (13) (11)

In the above formula, $A^5$, $A^6$, $R^5$, $X^3$ and $M^3$ represent the same groups as defined above.

Examples of the compound represented by formula (12) include alkanolamine, monoalkylalkanolamine and the like.

In formula (13), $X^3$ represents a fluorine atom, a chlorine atom, a bromine atom or an iodine atom.

In formula (13), examples of the cation represented by $M^3$ include sodium ion, potassium ion and the like.

In the practice of the reaction of the present invention, 1 to 5 moles, preferably 1 to 2 moles, of the compound (13) is allowed to react with 1 mole of the compound (12). The above reaction may be carried out at a temperature of from 20 to 120 °C, preferably from 40 to 90 °C in the presence of an inert solvent.

Examples of the inert solvent to be used in this reaction include polar solvents such as water, methanol, ethanol, isopropyl alcohol, dimethylformamide, dimethyl sulfoxide and the like and a mixture of two or more of these solvents, of which a lower alcohol alone or a mixture of water and a lower alcohol is particularly preferred.

In addition to the compound of interest represented by formula (11), the reaction product thus obtained contains inorganic salts as by-products and unreacted amine compound and the compound of formula (13) and/or hydrolyzed products thereof. Ratio of each component in the reaction product depends on the types of materials to be used, their reaction ratios, types and amounts of solvents to be used, reaction temperatures and the like. In consequence, the reaction product may be used as it is depending on the purpose, but, when a high purity product is required, it may be subjected to purification by usually used means such as solvent fractionation, ion exchange chromatography, electrodialysis and the like.

According to the carboxybetaine (14) of the present invention, $A^7$ is a straight- or branched-chain alkylene group having 3 to 36, preferably 3 to 18, carbon atoms. Illustrative examples of the alkylene group having 3 to 36 carbon atoms include propylene, butylene, pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, heptacosylene, octacosylene, nonacosylene, triacontylene, hentriacontylene, dotriacontylene, tritriacontylene, tetratriacontylene, pentatriacontylene, hexatriacontylene and the like groups.

In formula (14), $R^6$ and $R^7$ are the same or different from each other and each represents a hydrogen atom, a straight- or branched-chain alkyl group having 1 to 36 carbon atoms or an alkenyl group having 2 to 36 carbon atoms. Illustrative examples of the alkyl group include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, pentacosyl, hexacosyl, heptacosyl, octacosyl, triacontyl, hentriacontyl, dotriacontyl, tritriacontyl, tetratriacontyl, pentatriacontyl, hexatriacontyl and the like groups. Illustrative examples of the alkenyl group having 2 to 36 carbon atoms include vinyl, allyl, oleyl, palmitoleyl and the like groups. Of the above groups, hydrogen atom or methyl group is particularly preferred as $R^6$ or $R^7$.

The carboxybetaine (14) of the present invention can be produced for example in accordance with the following method 1 or 2.

Method 1:

In this method, the compound (14) of the present invention is obtained by allowing an amine compound (15) to react with a compound (16) in accordance with the following reaction formula.

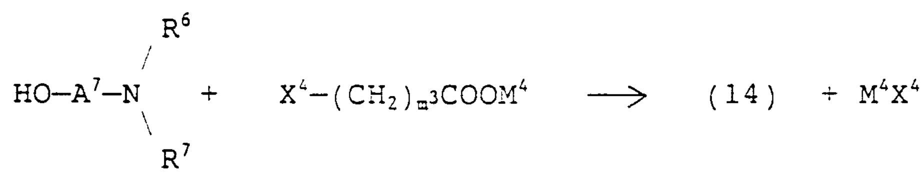

(15) (16)

In the above formula, $A^7$, $R^6$, $R^7$ and $m^3$ represent the same groups as defined above; $X^4$ represents a halogen atom and $M^4$ represents a hydrogen atom or a cationic group.

In this method, 1 to 5 moles, preferably 1 to 2 moles, of the compound (16) may be used based on 1 mole of the amine compound (15). In the formula of the compound (16), the halogen atom represented by $X^4$ may be selected from fluorine, chlorine, bromine and iodine, and illustrative examples of the cationic group of $M^4$ include alkali metal ions such as of sodium, potassium, lithium and the like and ammonium ion.

The above reaction may be carried out at a temperature of from 20 to 120 °C, preferably from 40 to 90 °C in an inert solvent. Examples of the inert solvent to be used in this reaction include polar solvents such as water, methanol, ethanol, isopropyl alcohol, dimethylformamide, dimethyl sulfoxide and the like and a mixture of two or more of these solvents, of which water alone or a mixture of water and a lower alcohol is particularly preferred, especially a water/lower alcohol mixture having a weight ratio of from 80/20 to 50/80 from a yield improving point of view.

Since the reaction product thus obtained contains unreacted materials and by-products, it may if necessary be subjected to purification by usually used means such as solvent fractionation, ion exchange chromatography, electrodialysis and the like.

Method 2:

In this method, the compound (14) of the present invention is obtained by allowing an amino acid derivative (17) to react with a compound (18) in accordance with the following reaction formula.

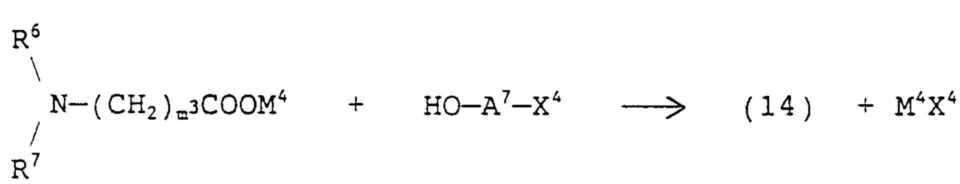

(17) (18)

In the above formula, $A^7$, $R^6$, $R^7$, $m^3$, $M^4$ and $X^4$ represent the same groups as defined above.

In the practice of this method, 1 to 3 moles, preferably 1 to 1.5 moles, of the compound (18) may be used based on 1 mole of the amino acid derivative (17). Other reaction conditions and purification method are the same as those described in the method 1.

In formula (19) representing the carboxybetaine of the present invention, $A^8$ represents a straight- or branched-chain alkylene group which may be substituted with a hydroxyl group and has 3 to 36, preferably 3 to 18, carbon atoms. Illustrative examples of the alkylene group having 3 to 36 carbon atoms include propylene, butylene, pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, heptacosylene, octacosylene, nonacosylene, triacontylene, hentriacontylene, dotriacontylene, tritriacontylene, tetratriacontylene, pentatriacontylene, hexatriacontylene and the like groups, which may be substituted with a hydroxyl group. Of these, alkylene groups having 3 to 12 carbon atoms are particularly preferred.

In formula (19), $R^8$ and $R^9$ are the same or different from each other and each represents a straight- or branched-chain alkyl group having 1 to 36 carbon atoms or an alkenyl group having 2 to 36 carbon atoms. Illustrative examples of the alkyl group include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, pentacosyl, hexacosyl, heptacosyl, octacosyl, triacontyl, hentriacontyl, dotriacontyl, tritriacontyl, tetratriacontyl, pentatriacontyl, hexatriacontyl and the like groups. Illustrative examples of the alkenyl group having 2 to 36 carbon atoms include vinyl, allyl, oleyl, palmitoleyl and the like groups. Of the above groups, methyl group is particularly preferred as $R^8$ or $R^9$.

The carboxybetaine (19) of the present invention can be produced for example in accordance with the following reaction formula, by allowing an amine compound (20) to react with a compound (21).

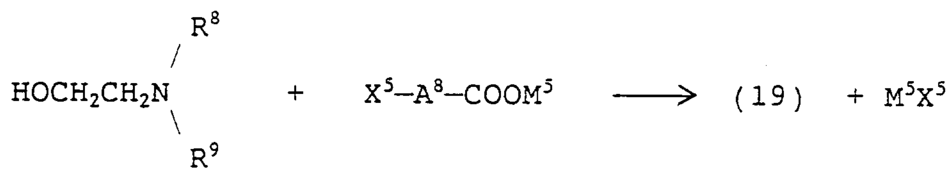

(20) (21)

In the above formula, $A^8$, $R^8$ and $R^9$ are the same groups as defined above; $X^5$ represents a halogen atom and $M^5$ represents hydrogen atom or a cationic group.

In this method, 1 to 5 moles, preferably 1 to 2 moles, of the compound (21) may be used based on 1 mole of the amine compound (20). In the formula of the compound (21), the halogen atom represented by $X^5$ may be selected from fluorine, chlorine, bromine and iodine, and illustrative examples of the cationic group of $M^5$ include alkali metal ions such as of sodium, potassium, lithium and the like and ammonium ion.

The above reaction may be carried out at a temperature of from 20 to 120 °C, preferably from 40 to 90 °C in an inert solvent. Examples of the inert solvent to be used in this reaction include polar solvents such as water, methanol, ethanol, isopropyl alcohol, dimethylformamide, dimethyl sulfoxide and the like and a mixture of two or more of these solvents, of which water alone or a mixture of water and a lower alcohol is particularly preferred, especially a water/lower alcohol mixture having a weight ratio of from 80/20 to 50/80 from a yield improving point of view.

Since the reaction product thus obtained contains unreacted materials and by-products, it may if necessary be subjected to purification by usually used means such as solvent fractionation, ion exchange chromatography, electrodialysis and the like.

In formula (22) representing the carboxybetaine of the present invention, illustrative examples of the alkyl group having 1 to 5 carbon atoms represented by $R^{10}$ and $R^{11}$ include methyl, ethyl, straight- or branched-chain propyl, butyl, pentyl and the like groups, and illustrative examples of the straight- or branched-chain alkyl group having 1 to 5 carbon atoms and containing a hydroxy group include the above-mentioned alkyl groups substituted with a hydroxyl group, such as 1-hydroxyethyl, 1-hydroxypropyl, 2-hydroxypropyl, 1,2-dihydroxypropyl, 1-hydroxybutyl, 1-hydroxypentyl and the like groups. Of these $R^{10}$ and $R^{11}$ groups, methyl, 1-hydroxyethyl, 1-hydroxypropyl and 1,2-dihydroxypropyl are particularly preferred. In this instance, at least one of $R^{10}$ and $R^{11}$ is an alkyl group containing a hydroxy group.

In formula (22), $X^6$ represents a hydrogen atom or a cation. Examples of the cation include alkali metals, alkaline earth metals, amines, basic amino acids, quaternary ammonium compounds and the like, more illustratively, sodium, potassium, magnesium, calcium, ethanolamine, methylethanolamine, dimethylethanolamine, diethanolamine, methyldiethanolamine, triethanolamine, lysine, arginine, choline and the like.

The carboxybetaine (22) of the present invention can be produced for example in accordance with the following reaction formula:

$$\underset{(23)}{R^{10}-\underset{H}{N}-R^{11}} + \underset{(24)}{2Y^1-CH_2-COOX^6} \longrightarrow (22)$$

wherein $R^{10}$, $R^{11}$ and $X^6$ represent the same groups as defined above; and $Y^1$ represents a halogen atom.

That is, the carboxybetaine (22) of interest is obtained by allowing an amine compound represented by formula (23) to react with a carboxylate represented by formula (24) in the presence of an inert solvent and the like, if necessary further carrying out salt exchange.

Illustrative examples of the compound (23) to be used as a starting material include monoalkylalkalolamines, dialkanolamines and the like, such as 2-(methylamino)ethanol, diethanolamine, 2-(ethylamino)-ethanol, 2-(propylamino)ethanol and the like.

In the compound (24) represented by the aforementioned formula (24) to be used as the other starting material, the halogen atom represented by $Y^1$ may be selected from fluorine, chlorine, bromine and iodine. Illustrative examples of the compound (24) include sodium chloroacetate, sodium bromoacetate, chloroacetic acid and the like.

The above reaction may be carried out using 2 to 10 moles, preferably 2 to 4 moles, of the compound (24) based on 1 mole of the compound (23), at a temperature of from 20 to 120 °C, preferably from 40 to 90 °C in the presence of an inert solvent.

Examples of the inert solvent to be used in this reaction include polar solvents such as water, methanol, ethanol, isopropyl alcohol, dimethylformamide, dimethyl sulfoxide and the like and a mixture of two or more of these solvents, of which a lower alcohol alone or a mixture of water and a lower alcohol is particularly preferred.

Also, in order to obtain high reaction efficiency, it is desirable to use a base in the reaction system in an amount of from 1 to 3 moles based on 1 mole of the compound (23). Examples of such bases include sodium hydroxide, potassium hydroxide and the like.

In addition to the carboxybetaine (22) of interest, the reaction product thus obtained contains inorganic salts as by-products and unreacted amine compound and the compound represented by formula (24) and/or hydrolyzed products thereof. Ratio of each component in the reaction product depends on the types of materials to be used, their reaction ratios, types and amounts of solvents to be used, reaction temperatures and the like. In consequence, the reaction product may be used as it is depending on the purpose, but, when a high purity product is required, it may be subjected to purification by usually used means such as solvent fractionation, ion exchange chromatography, electrodialysis and the like.

When $X^6$ in formula (22) is not hydrogen atom but a cation, the cation in the thus obtained carboxybetaine (22) can be exchanged for hydrogen atom making use of an ion exchange chromatography. Thereafter, salt exchange of $X^6$ may be made easily by neutralizing the compound with a desired base.

In the group $R^{12}$ of the aforementioned formula (25) representing the carboxybetaine of the present invention, illustrative examples of the alkyl group having 1 to 6 carbon atoms include methyl, ethyl, propyl, isopropyl, butyl, t-butyl, pentyl, hexyl and the like groups, and illustrative examples of the hydroxyl group-substituted alkyl group having 1 to 6 carbon atoms include hydroxymethyl, 1-hydroxyethyl, 1-hydroxypropyl, 2-hydroxyethyl, 1,2-hydroxypropyl, 1-hydroxybutyl, 1-hydroxypentyl, 1-hydroxyhexyl and the like groups. Of these alkyl groups having 1 to 6 carbon atoms, which may be substituted with a hydroxyl group, methyl group, ethyl group, hydroxymethyl group, 1-hydroxyethyl group and 2-hydroxyethyl group are preferred.

When $R^{12}$ is a group represented by $-N(R^{14})(R^{15})$, $R^{14}$ and $R^{15}$ are the same or different and each represents hydrogen atom or an alkyl groups having 1 to 6 carbon atoms which may be substituted with a hydroxyl group, and examples of these 1 to 6 carbon alkyl groups which may be substituted with a hydroxyl group include those mentioned for $R^{12}$. Among these $R^{14}$ and $R^{15}$ groups, hydrogen atom, methyl group

and 1-hydroxyethyl group are particularly preferred.

Among the $R^{13}$ groups, examples of the 1 to 6 carbon alkyl group which may be substituted with a hydroxyl group include those mentioned for $R^{12}$. Of these $R^{13}$ groups, hydrogen atom and methyl group are particularly preferred.

The carboxybetaine (25) of the present invention can be produced for example in accordance with the following reaction formula in which an amine compound represented by formula (26) is allowed to react with a haloacetic acid or a salt thereof represented by formula (27).

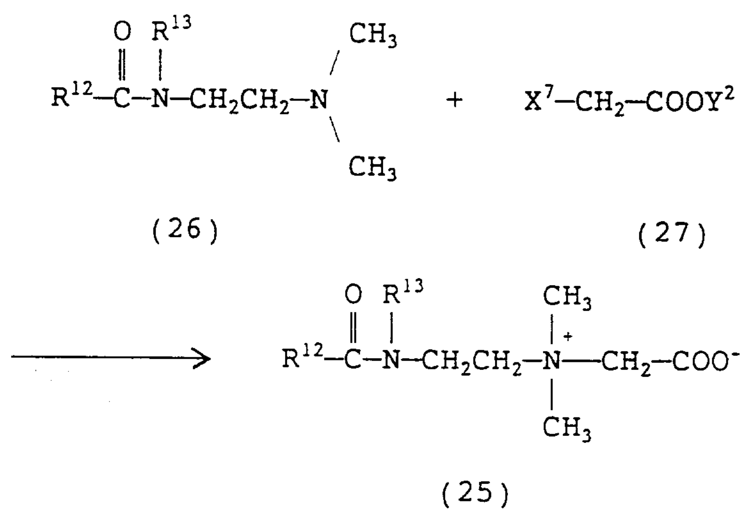

(26) (27)

(25)

In the above formula, $R^{12}$ and $R^{13}$ represent the same groups as defined above; $X^7$ represents a halogen atom and $Y^2$ represents hydrogen atom or a cation.

In the production process of the present invention, the amine compound (26) to be used as a starting material may be obtained in accordance with the following reaction formula (A) in which a carboxylic acid compound represented by formula (28) is allowed to react with an amine compound represented by formula (29) in an inert solvent, or in accordance with the following reaction formula (B) in which urea or a urea derivative represented by formula (30) is allowed to react with N,N-dimethylethanolamine represented by formula (31) in an inert solvent.

Reaction formula (A):

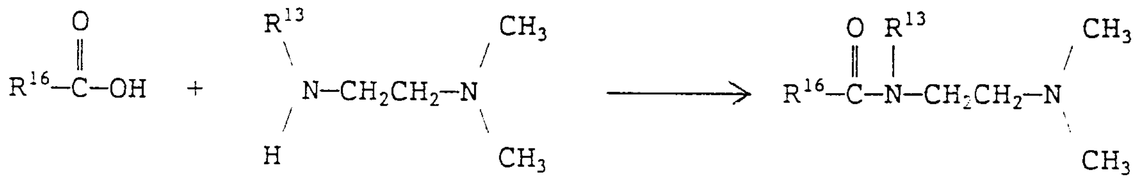

(28) (29) (26')

Reaction formula (B):

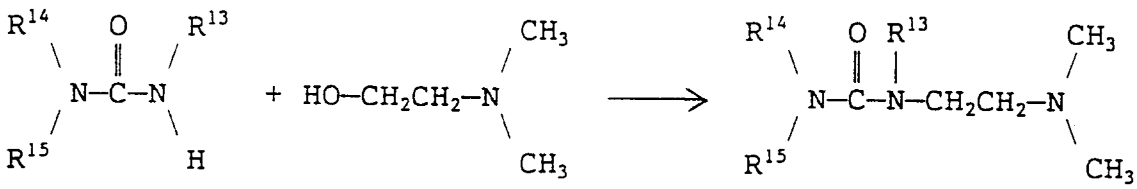

(30) (31) (26")

In the above formulae, $R^{13}$, $R^{14}$ and $R^{15}$ represent the same groups as defined above; and $R^{16}$ represents an alkyl group having 1 to 6 carbon atoms which may be substituted with a hydroxyl group, or a group represented by the following chemical structure.

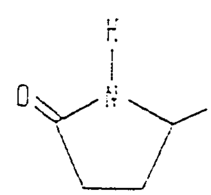

In the above reaction formula (A), examples of the carboxylic acid compound (28) include acetic acid, propionic acid, butanoic acid, pentanoic acid, hexanoic acid, glycolic acid, lactic acid, glyceric acid, pyrrolidonecarboxylic acid and the like, and examples of the amine compound (29) include N,N-dimethylethylenediamine, N,N-dimethyl-N'-methylethylenediamine and the like.

The reaction of the above reaction formula (A) may be carried out using 0.5 to 3 moles, preferably 1.0 to 1.5 moles, of the amine compound (29) based on 1 mole of the carboxylic acid compound (28), at a temperature of from 40 to 200 °C, preferably from 80 to 150 °C, in an inert solvent.

Examples of the inert solvent to be used in this reaction include those having low compatibility with water, such as toluene, xylene and the like. From the viewpoint of improving reaction efficiency, it is desirable to trap formed water during the reaction.

In the above reaction formula (B), examples of the urea derivative (30) include methylurea, ethylurea, 2-hydroxyethylurea and the like.

The reaction of the above reaction formula (B) may be carried out using 0.5 to 5 moles, preferably 1.0 to 2.0 moles, of urea or the urea derivative (30) based on 1 mole of N,N-dimethylethanolamine (31), at a temperature of from 40 to 150 °C, in an acid-containing inert solvent.

Examples of the acid to be used in this reaction include hydrochloric acid, acetic acid, sulfuric acid and the like and a mixture of two or more acids selected therefrom, and examples of the inert solvent include polar solvents such as water, methanol, ethanol, isopropyl alcohol, dimethyl sulfoxide, dimethylformamide, and the like and a mixture of two or more of these solvents, of which water alone or a mixture of water and a lower alcohol is particularly preferred.

In formula (27) representing a haloacetic acid or a salt thereof to be used as the other starting material in the production process of the present invention, examples of the halogen atom represented by $X^7$ include fluorine, chlorine, bromine, iodine and the like atoms. Also, examples of the cation represented by $Y^2$ include sodium ion, potassium ion and the like. Examples of such haloacetic acids or their salts include sodium chloroacetate, sodium bromoacetate, chloroacetic acid and the like.

The production process of the present invention is effected by allowing the amine compound (26) obtained in accordance with the above reaction formula (A) or (B) to react with the haloacetic acid or a salt thereof (27), which may be carried out using 1 to 5 moles, preferably 1 to 2 moles, of the haloacetic acid or a salt thereof (27) based on 1 mole of the amine compound (26), in an inert solvent at a temperature of from 20 to 120 ° C, preferably from 40 to 90 ° C.

Examples of the inert solvent to be used in this reaction include polar solvents such as water, methanol, ethanol, isopropyl alcohol, dimethylformamide, dimethyl sulfoxide and the like and a mixture of two or more of these solvents, of which water alone or a mixture of water and a lower alcohol is particularly preferred.

Also, in order to obtain high reaction efficiency, it is desirable to use a base in the reaction system in an amount of from 1 to 3 moles based on 1 mole of the amine compound (26). Examples of such bases include sodium hydroxide, potassium hydroxide and the like.

In addition to the carboxybetaine (25) of interest, the reaction product thus obtained contains inorganic salts as by-products and unreacted amine compound (26) and haloacetic acid or a salt thereof (27) and/or hydrolyzed products thereof. Ratio of each component in the reaction product depends on the types of materials to be used, their reaction ratios, types and amounts of solvents to be used, reaction temperatures and the like. In consequence, the reaction product may be used as it is depending on the purpose, but, when a high purity product is required, it may be subjected to purification by usually used means such as solvent fractionation, ion exchange chromatography, electrodialysis and the like.

In formula (32), preferred examples of $Z^3$ include 2,3-dihydroxypropyl group, 2,6,7-trihydroxy-4-oxaheptyl group, a polyglycerol group represented by:

$$HOCH_2CH(OH)CH_2O{-}(CH_2CH(OH)CH_2O)_{m3}{-}CH_2CH(OH)CH_2{-}$$

($m^3$ represents an integer of 1 to 10), 2-hydroxyethyl group, 3-hydroxypropyl group, 4-hydroxybutyl group, 5-hydroxypentyl group and the like.

In formula (32), preferred examples of each of $R^{17}$ and $R^{18}$ include methyl, ethyl, propyl, butyl, pentyl and the like groups.

In formula (32), preferred examples of $Y^3$ include methylene, ethylene, trimethylene, tetramethylene, pentamethylene, hydroxyethylene, hydroxymethylmethylene and the like groups.

Particularly preferred examples of the carboxybetaine (32) to be used in the present invention are those which satisfy the following formula.

$$\frac{\left[31 \times \begin{pmatrix}\text{Number of primary}\\ \text{hydroxy group}\\ \text{in one molecule}\end{pmatrix} + 51 \times \begin{pmatrix}\text{Number of carboxy-}\\ \text{betaine group}\\ \text{in one molecule}\end{pmatrix}\right]}{(\text{Molecular weight})} \geqq 0.20$$

Illustrative examples of the compounds include those represented by the following formulae (32-1) to (32-8).

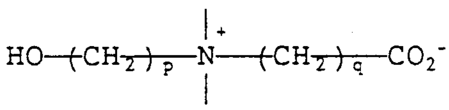

(32-1)

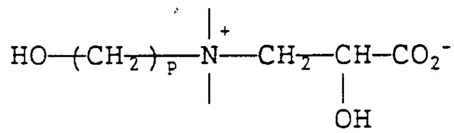

(32-2)

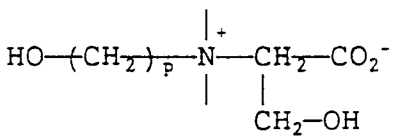

(32-3)

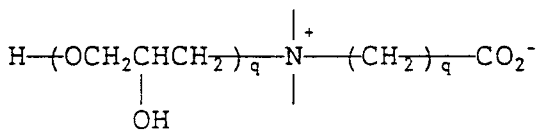

(32-4)

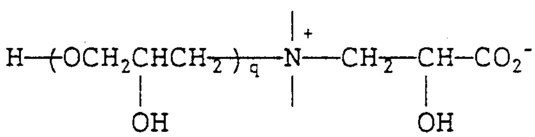

(32-5)

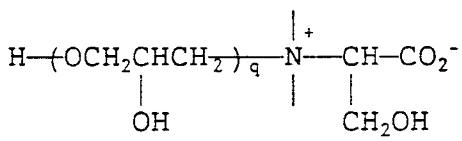

(32-6)

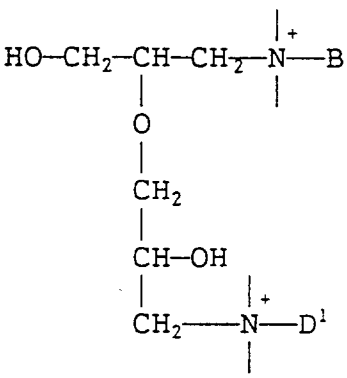

(32-7)

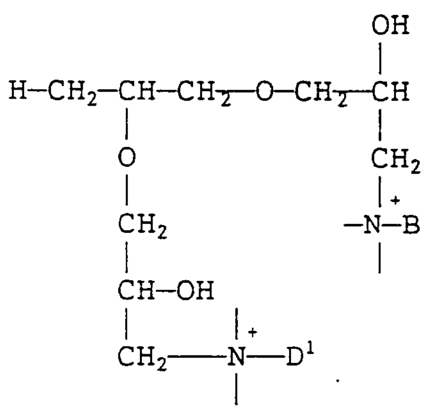

(32-8)

In the above formulae, p represents an integer of 2 to 5, q represents an integer of 1 or 2, and B and $D^1$ are the same or different and each represents $—CH_2—CO_2^-$, $—CH_2\text{-}CH_2\text{-}CO_2^-$ or

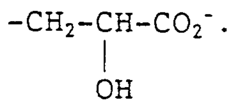

Of these, particularly preferred carboxybetaine is a compound of the above formula (32-1) in which p is 2 and q is 1.

The carboxybetaine (32) of the present invention may be produced for instance in accordance with the following reaction formula.

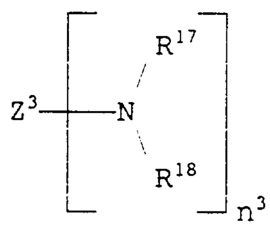 + $n^3 \cdot X^8-Y^3-COOM^6$

(33) (34)

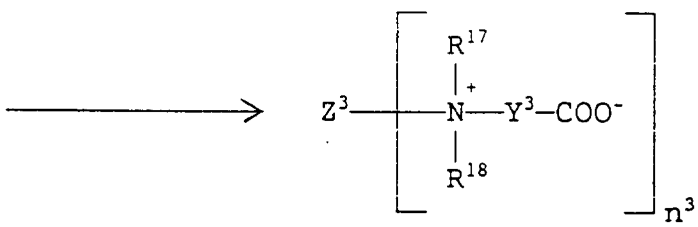 + $n^3 \cdot M^6X^8$

In the above formula, $X^8$ represents a halogen atom, $M^6$ represents a cation, and $Z^3$, $R^{17}$, $R^{18}$, $Y^3$ and $n^3$ are as defined above.

Preferred examples of the compound represented by formula (33) include a compound which is obtained by allowing glycidol, glycerol or a glycerol condensate to react with an epihalohydrin and then allowing the thus formed glycidyl-etherified glycerol or glycerol condensate to react with a dialkylamine, as well as a dialkylalkanolamine and the like.

In formula (34), examples of $X^8$ includes fluorine, chlorine, bromine, iodine and the like halogen atoms, and examples of $M^6$ includes $Na^+$, $K^+$ and the like cations.

The above reaction may be effected by allowing 1 mole of the compound (33) to react with 1 to 5 moles, preferably 1 to 2 moles, of the compound (34) at a temperature of from 20 to 120 °C, preferably from 40 to 90 °C, in the presence of an inert solvent.

Examples of the inert solvent to be used in the above reaction include polar solvents such as water, methanol, ethanol, isopropanol, dimethylformamide, dimethyl sulfoxide and the like and a mixture of two or more of these solvents, of which lower alcohols and a mixture of water with a lower alcohol are particularly preferred.

In the aforementioned formula (35), illustrative examples of $A^{10}$ include ethylene, propylene, butylene, pentylene, hexylene, heptylene, octylene, nonylene, decylene, undecylene, dodecylene and the like groups. These groups may be substituted with a hydroxyl group. Of these alkylene groups, a group having 2 to 6 carbon atoms are particularly preferred.

In the aforementioned formula (35), illustrative examples of the alkyl group represented by $R^{19}$ and $R^{20}$ include methyl, ethyl, propyl, butyl, pentyl, hexyl, heptyl, octyl, nonyl, decyl, undecyl, dodecyl and the like groups, and illustrative examples of the alkenyl groups include vinyl, allyl and the like groups. These groups may be substituted with a hydroxyl group. Of these groups, methyl group is particularly preferred.

The sulfobetaine (35) of the present invention can be produced by allowing a compound represented by the following formula (36):

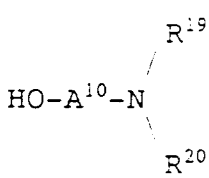 (36)

wherein $A^{10}$, $R^{19}$ and $R^{20}$ represent the same group as defined above,
to react with a compound represented by the following formula (37):

$$X^9—CH_2—\underset{\displaystyle OH}{\underset{|}{CH}}—CH_2—SO_3M^7 \qquad (37)$$

wherein $X^9$ represents a halogen atom and $M^7$ represents a hydrogen atom or a cation.

In the production process of the sulfobetaine (35) of the present invention, it is preferable to allow 1 mole of the compound (36) to react with 1 to 5 moles, preferably 1 to 2 moles of the compound (37).

In formula (37), examples of the halogen atom represented by $X^9$ include fluorine, chlorine, bromine, iodine and the like, and cations represented by $M^7$ include alkali metal ions such as sodium, potassium, lithium and the like and ammonium ion.

The above reaction may be carried out at a temperature of from 20 to 120 °C, preferably from 40 to 90 °C, in the presence of an inert solvent. Examples of the inert solvent to be used in the above reaction include polar solvents such as water, methanol, ethanol, isopropyl alcohol, dimethylformamide, dimethyl sulfoxide and the like and a mixture of two or more of these solvents, of which water alone or a mixture of water and a lower alcohol, especially having a water/lower alcohol weight ratio of 80/20 to 50/80, is particularly preferred from a yield improving point of view.

Since the reaction product thus formed contains unreacted compounds, by-products and the like, it may if necessary be subjected to purification by usually used means such as solvent fractionation, ion exchange chromatography, electrodialysis and the like.

Since the thus obtained carboxybetaines and sulfobetaine according to the present invention have an excellent moisture keeping ability, not only it can be used as a moisture keeping agent as a matter of course, but also it can be applied to various types of skin and hair cosmetics, as well as detergents such as shampoos, kitchen detergents and the like. In particular, the compound of the present invention is employed in a skin or hair cosmetic, it exerts an excellent moisture keeping ability which is hardly spoiled by perspiration, and it does not cause sticky feel but provides the skin or hair with a moist feel.

A carboxybetaine represented by the following formula (14'):

$$HO—A^{7'}—\overset{\displaystyle R^{6'}}{\overset{|}{\underset{\displaystyle R^{7'}}{\underset{|}{N^+}}}}—(CH_2)_{m3}COO^- \qquad (14')$$

wherein $A^{7'}$ represents a straight- or branched-chain alkylene group having 2 to 36 carbon atoms; $R^{6'}$ and $R^{7'}$ are the same or different and each represents hydrogen atom, a straight- or branched-chain alkyl group having 1 to 36 carbon atoms or an alkenyl group having 2 to 36 carbon atoms each of which may be substituted with a hydroxyl group; and $m^3$ represents an integer of 1 or 2,
which may be produced in the same manner as the carboxybetaine (14), has also an excellent moisture keeping ability as the carboxybetaine and sulfobetaine of the present invention, thus it can also be employed as an embodiment of the present invention.

The cosmetic of the present invention may contain the afore-mentioned carboxybetaine or the sulfobetaine in an amount of from 0.1 to 20 % by weight, preferably from 0.5 to 10 % by weight.

In addition to the carboxybetaine or the sulfobetaine according to the present invention, the cosmetic of the present invention may be blended with other usually used cosmetic components as a cosmetic base (component (b)) within such a range that they do not spoil effects of the present invention. Examples of such components include: polyhydric alcohols such as ethylene glycol, diethylene glycol, triethylene glycol, more higher degree polyethylene glycols, propylene glycol, 1,3-propanediol, dipropylene glycol, more higher degree polypropylene glycols, butylene glycols including 1,3-butylene glycol, 1,4-butylene glycol, isobutylene glycol and the like, glycerol, diglycerol, more higher degree polyglycerols, sugar alcohols including sorbitol, mannitol, xylitol, maltitol and the like, ethylene oxide (to be referred to as "EO" hereinafter) and propylene oxide (to be referred to as "PO" hereinafter) addition products of glycerols, EO and PO addition products of sugar alcohols, EO and PO addition products of galactose, glucose, fructose and the like monosaccharides and EO and PO addition products of maltose, lactose and the like polysaccharides; oil components including hydrocarbons such as liquid paraffin, squalane, vaseline, solid paraffin and the like, natural oils and fats such as olive oil, jojoba oil, evening primrose oil, coconut oil, beef

tallow and the like, ester oils such as isopropyl myristate, cetylisooctanoate, neopentyl glycol dicaprate and the like, silicone oils including dimethylsilicone, methylphenylsilicone, cyclic silicones and the like and higher fatty acids such as isostearic acid, oleic acid and the like; surfactants including nonionic surfactants such as polyoxyethylene (to be referred to as "POE" hereinafter) alkyl ether, POE branched alkyl ether, POE sorbitan ester, POE glycerol fatty acid ester, POE hydrogenated castor oil, sorbitan ester, glycerol fatty acid ester, polyglycerol fatty acid ester and the like, anionic surfactants such as of phosphate base, sulfonate base, sulfate base, carbonate base and the like, as well as ampholytic and cationic surfactants; drugs including vitamins, germicides such as triclosan, trichlorocarban and the like, anti-inflammatory agents such as glycyrrhizin dipotassium salt, tocopherol acetate and the like, anti-dandruff agents such as zinc pyrithion, octopyrox and the like, activators, cooling agents such as menthol and the like, and UV absorbents; antiseptics such as methylparaben, butylparaben and the like, foaming agents such as alkylamine oxide, fatty acid alkanolamine and the like, viscosity adjusting agents such as inorganic salts, polyethylene glycol stearate, ethanol and the like, pearling agents, perfumes, coloring matters, antioxidants and the like; water swelling clay minerals such as montmorillonite, saponite, hectorite, beegum, knibia, smecton and the like; high molecular compounds including polysaccharides such as carrageenan, xanthan gum, sodium alginate, pullulan, methyl cellulose, carboxymethyl cellulose, hydroxyethyl cellulose, hydroxypropyl cellulose and the like and synthetic high polymers such as carboxyvinyl polymers, polyvinyl pyrrolidone and the like; and pigments including body pigments such as titanium oxide, kaolin, mica, sericite, zinc oxide, talc and the like and high polymer powders such as polymethyl methacrylate, nylon powder and the like.

The cosmetic of the present invention can be produced by the usually used means, with optional preparation forms such as liquid, cream, paste, solid, powder and the like, of which liquid, cream or paste form is particularly preferred.

Specific and non-limiting examples of the cosmetic according to the present invention include a face lotion, a cosmetic emulsion, a cosmetic cream, a pack, a foundation, a lipstick, a mascara, a nail enamel, a shampoo, a body shampoo, a rinse, a body rinse and the like.

The detergent composition of the present invention may contain the afore-mentioned carboxybetaine or the sulfobetaine in an amount of from 0.5 to 50 % by weight, preferably from 1 to 30 % by weight.

Various types of surfactants which are generally used in detergents may be used optionally in the detergent composition of the present invention as component (c), within such a range that they do not spoil effects of the present invention.

Illustrative examples of the anionic surfactant include: sulfate and sulfonate base surfactants such as of alkyl sulfate, polyoxyethylene alkyl sulfate, sulfosuccinate, taurate, isothionate, a-olefin sulfonate and the like surfactants; carboxylate base surfactants such as fatty acid soap, an ether carboxylate base surfactant, an acylated amino acid base surfactant and the like; and phosphate base surfactants such as an alkyl phosphate base surfactant and the like.

Illustrative examples of the ampholytic surfactant include carboxybetaine base, phosphobetaine base, sulfobetaine base, imidazoliniumbetaine base and the like surfactants.

Illustrative examples of the nonionic surfactant include a polyoxyalkylene addition type, a polyoxypropylene-polyoxyethylene addition type, an amine oxide base, mono- or diethanolamide base and the like, as well as polyhydric alcohol types such as a sorbitan fatty acid ester, a glycerol fatty acid ester, a sucrose fatty acid ester, an alkylsaccharide base, an N-polyhydroxyalkylfatty acid amide base and the like.

Illustrative examples of the cationic surfactant include a mono- or dialkyl addition type quaternary ammonium salt having a straight- or branched-chain alkyl group, and its derivative in which an alkylene oxide is further added to the alkyl group, of which a straight-chain monoalkyl quaternary ammonium salt having 12 to 16 carbon atoms, a quaternary ammonium salt having a branched alkyl group of 20 to 28 carbon atoms are particularly preferred.

These surfactants may be used alone or as a mixture of two or more in the detergent composition of the present invention in an amount of from 2 to 60 % by weight, preferably from 10 to 50 % by weight, though it varies depending on the preparation form. Further, the surfactant may be used at a weight ratio of from 1:2 to 1:50, preferably from 1:3 to 1:30, based on the carboxybetaine or the sulfobetaine of the present invention.

In addition to the above components, the detergent composition of the present invention may be blended optionally with other usually used detergent components within such a range that they do not spoil effects of the present invention. Examples of such components include: polyhydric alcohols such as ethylene glycol, diethylene glycol, triethylene glycol, more higher degree polyethylene glycols, propylene glycol, 1,3-propanediol, dipropylene glycol, more higher degree polypropylene glycols, butylene glycols including 1,3-butylene glycol, 1,4-butylene glycol, isobutylene glycol, and the like, glycerol, diglycerol, more

higher degree polyglycerols, sugar alcohols including sorbitol, mannitol, xylitol, maltitol and the like, EO or PO addition products of glycerols, EO or PO addition products of sugar alcohols, EO or PO addition products of galactose, glucose, fructose and the like monosaccharides and EO or PO addition products of maltose, lactose and the like polysaccharides; oil components including hydrocarbons such as liquid paraffin, squalane, vaseline, solid paraffin and the like, natural oils such as olive oil, jojoba oil, evening primrose oil, coconut oil, beef tallow and the like, ester oils such as isopropyl myristate, cetylisooctanoate, neopentyl glycol dicaprate and the like, silicone oils such as dimethylsilicone, methylphenylsilicone, cyclic silicones and the like and higher fatty acids such as isostearic acid, oleic acid and the like; drugs including vitamins, germicides such as triclosan, trichlorocarban and the like, anti-inflammatory agents such as glycyrrhizin dipotassium salt, tocopherol acetate and the like, anti-dandruff agents such as zinc pyrithion, octopyrox and the like, activators, cooling agents such as menthol and the like, and UV absorbents; water swelling clay minerals such as montmorillonite, saponite, hectorite, beegum, knibia, smecton, and the like; high molecular compounds including polysaccharides such as carrageenan, xanthan gum, sodium alginate, pullulan, methyl cellulose, carboxymethyl cellulose, hydroxyethyl cellulose, hydroxypropyl cellulose and the like and synthetic high polymers such as carboxyvinyl polymers, polyvinyl pyrrolidone and the like; pigments including body pigments such as titanium oxide, kaolin, mica, sericite, zinc oxide, talc and the like and high polymer powders such as polymethyl methacrylate, nylon powder and the like; antiseptics such as methylparaben, butylparaben and the like; viscosity adjusting agents such as inorganic salts, polyethylene glycol stearate, ethanol and the like; pearling agents; perfumes; coloring matters; and antioxidants.

The detergent composition of the present invention can be produced by the usually used means, with optional preparation forms such as liquid, paste, solid, powder and the like, of which liquid or paste form is particularly preferred.

Since the carboxybetaine and the sulfobetaine of the present invention have an excellent moisture keeping ability, it can be applied to detergent compositions such as shampoos, as well as rinses and various types of cosmetics. In addition, the compound of the present invention can be produced easily at a low cost using easily available materials.

The following examples are provided to further illustrate the present invention. It is to be understood, however, that the examples are for purpose of illustration only and are not to be construed to limit the scope of the invention.

## EXAMPLE 1

Production of carboxybetaine (1):

A reactor was charged with 900 g (10.2 mol) of 50 % by weight aqueous solution of dimethylamine, 126 g (7.6 mol) of 2,3-dihydroxypropyl 2,3-epoxypropyl ether added thereto in a dropwise manner over 3 hours at room temperature. After completion of the dropwise addition, temperature of the resulting mixture was gradually elevated to 50 °C, and the reaction was carried out for 4 hours at the same temperature. After completion of the reaction, remaining dimethylamine and water were removed under a reduced pressure to obtain 1482 g of a crude product which was subsequently dissolved in 1,000 g of water. While keeping the reaction system at 60 °C, and 1,000 g (8.7 mol) of sodium chloroacetate dissolved in 1,000 g of water was added thereto in a dropwise manner over 5 hours, followed by 5 hours of reaction at 60 °C. After completion of the reaction, the resulting reaction solution was directly subjected to purification by an ion exchange chromatography (ion exchange resin: AG501-X8 manufactured by Bio-Rad Laboratories, Inc.), followed by distillation removal of the solvent under reduced pressure to obtain 1,241 g of 1-carboxy-N,N-dimethyl-N-(2,6,7-trihydroxy-4-oxaheptyl)-methaneaminium hydroxide inner salt with a yield of 65 %. This compound was a hygroscopic greasy colorless solid which showed a purity of 99 % when analyzed by HPLC (column: Shimadsu Gel SCR101N manufactured by Shimadzu Corp.; eluent: water).

$^1$H-NMR ($D_2O$): $\delta$ (ppm) (Fig. 1)
3.19 (singlet, 6H, a)
3.30 - 3.60 (complex multiplet, 6H, b + c + d)
3.61 - 3.71 (doublet, 2H, g)
3.72 - 3.83 (quintet, 1H, e)
3.84 - 3.93 (singlet, 2H, h)
4.18 - 4.35 (quintet, 1H, f)

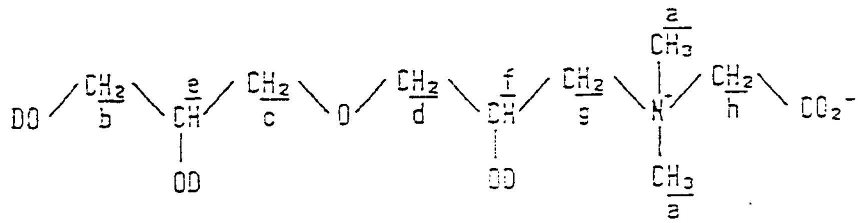

Mass spectrometry (FAB ionization method)
M/Z: 252 $(M+H)^+$ ($M = C_{10}H_{21}O_6N$)

EXAMPLE 2

Production of carboxybetaine (1):

A reactor was charged with 208 g (2.4 mol) of 50 % by weight aqueous solution of dimethylamine, and 200 g (0.4 mol) of an epichlorohydrine addition product (average addition mol numbers: 3) of a polyglycerol (average condensation degree: 4) was added thereto in a dropwise manner over 2 hours at room temperature. After completion of the dropwise addition, temperature of the resulting mixture was gradually elevated to 50 °C, and the reaction was carried out for 6 hours at the same temperature. After completion of the reaction, remaining dimethylamine and water were removed under reduced pressure to obtain 253.8 g of a crude product which was subsequently dissolved in 250 g of water. To the reaction system kept at 60 °C was added 190 g (1.6 mol) of sodium chloroacetate dissolved in 200 g of water, in a dropwise manner over 5 hours, followed by 5 hours of reaction at 60 °C. After completion of the reaction, the resulting reaction solution was directly subjected to purification by an ion exchange chromatography (ion exchange resin: AG501-X8 manufactured by Bio-Rad Laboratories, Inc.) to obtain 208 g of a carboxybetaine compound of a polyglycerol (average condensation degree: 4), as the compound of interest with a yield of 64 %.

This compound contained 3 carboxybetaine groups in average per 1 mol of the polyglycerol.
$^1$H-NMR ($D_2O$): $\delta$ (ppm) (Fig. 2)
3.19 (singlet, 18H,

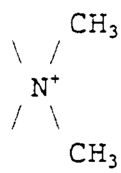
)

3.35 - 3.84 (complex multiplet, 32H, methylene and methine proton of polyglycerol skeleton,

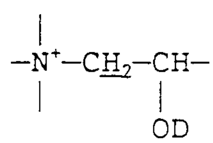
)

3.88 - 3.91 (singlet, 6H,

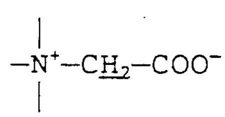
)

4.21 - 4.27 (broad multiplet, 3H,

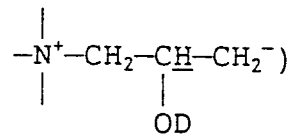

EXAMPLE 3

Production of carboxybetaine (6):

A reactor was charged with 36.5 g (0.31 mol) of methylhexylamine, and 300 g (2.0 mol) of 2,3-dihydroxypropyl 2,3-epoxypropyl ether was added thereto in a dropwise manner over 5 hours at room temperature.

After completion of the dropwise addition, temperature of the resulting mixture was gradually elevated to 50 °C, and the reaction was carried out for 4 hours at the same temperature. To the reaction system kept at 50 °C was added 54.1 g (0.47 mol) of sodium chloroacetate dissolved in 50 g of water, in a dropwise manner over 2 hours, followed by 6 hours of reaction at 50 °C. After completion of the reaction, the resulting reaction solution was concentrated under reduced pressure and then mixed with 1.3 l of ethanol to remove ethanol-insoluble materials by filtration. Thereafter, the thus obtained ethanol-soluble fraction was concentrated under reduced pressure and subjected to purification by a silica gel column chromatography (eluting solvent: chloroform/methanol) until a single spot was obtained by a thin layer chromatography, thereby obtaining 24.9 g of 1-carboxy-N-methyl-N-hexyl-N-(2,6,7-trihydroxy-4-oxaheptyl)-methaneaminium hydroxide inner salt with a yield of 25 %.

$^1$H-NMR ($D_2O$): $\delta$ (ppm)
0.75 (t, 3H, a)
1.21 (broad, 6H, b)
1.60 (broad, 2H, c)
3.11 (s, 3H, d)
3.29 - 3.65 (broad m, 11H, e)
3.80 (s, 2H, f)
4.22 (broad m, 1H, g)

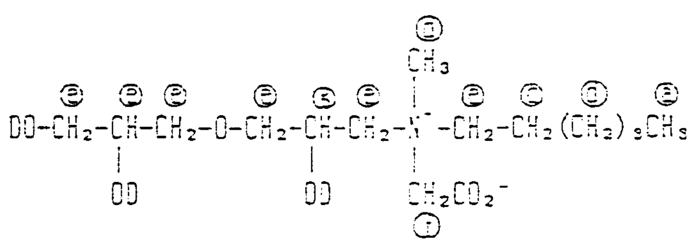

Mass spectrometry (FAB ionization method)
M/Z: 322 $(M+H)^+$ ($M = C_{15}H_{31}O_6N$)

EXAMPLE 4

Production of carboxybetaine (6):

A reactor was charged with 35.3 g (0.30 mol) of methylhexylamine, and 400 g (0.8 mol) of an epichlorohydrine addition product (average addition mol numbers: 3) of a polyglycerol (average condensation degree: 4) was added thereto in a dropwise manner over 5 hours at room temperature.

After completion of the dropwise addition, temperature of the resulting mixture was gradually elevated to 50 °C, and the reaction was carried out for 6 hours at the same temperature. To the reaction system kept at 50 °C was added 63.3 g (0.55 mol) of sodium chloroacetate dissolved in 100 g of water, in a dropwise manner over 2 hours, followed by 6 hours of reaction at 50 °C. After completion of the reaction, the resulting reaction solution was concentrated under a reduced pressure and then mixed with 1.5 l of ethanol to

remove ethanol-insoluble materials by filtration. Thereafter, ethanol was removed from the thus obtained ethanol-soluble fraction under a reduced pressure, and the residue was dissolved again in 2.0 l of water and subjected to purification by an ion exchange chromatography (ion exchange resin: AG501-X8 manufactured by Bio-Rad Laboratories, Inc.) to obtain 85.1 g of a carboxybetaine of a polyglycerol (average condensation degree: 4), as the compound of interest with a yield of 42 %. This compound contained 3 carboxybetaine groups in average per 1 mol of the polyglycerol.

$^1H$-NMR ($D_2O$): $\delta$ (ppm)

0.83 (t, 9H,

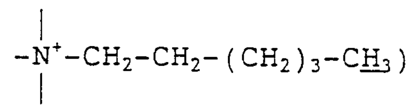

1.27 (broad, 18H,

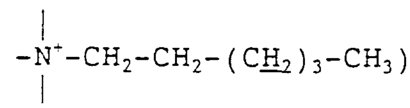

1.69 (broad, 6H,

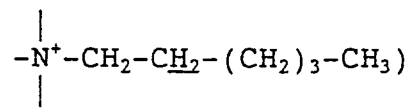

3.14 (s, 9H,

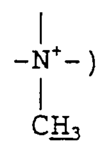

3.32 - 3.91 (broad m, 32H, methylene and methine proton of polyglycerol skeleton,

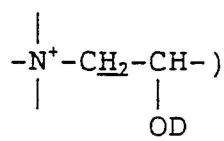

3.87 - 3.90 (s, 6H,

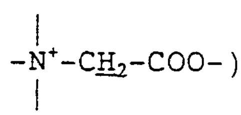

4.22 - 4.29 (broad m, 3H,

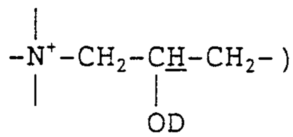

EXAMPLE 5

A reactor was charged with 130.5 g (1.0 mol) of sodium 3-chloropropionate and 300 g of ethanol. To this was subsequently added 42.8 g (0.7 mol) of 2-aminoethanol in a dropwise manner over 1 hour at room temperature.

After completion of the dropwise addition, temperature of the resulting mixture was gradually elevated to 80 ° C, and the reaction was carried out for 15 hours at the same temperature.
After completion of the reaction, the resulting reaction solution was directly subjected to purification by an ion exchange chromatography (ion exchange resin: AG501-X8 manufactured by Bio-Rad Laboratories, Inc.), followed by distillation removal of the solvent under reduced pressure to obtain 61.9 g of 3-(N-hydroxyethyl)-aminopropionic acid with a yield of 66 %.

This compound showed a purity of 99 % when analyzed by HPLC (column: Shimadzu Gel SCR101N manufactured by Shimadzu Corp.; eluent: water).

$^1$H-NMR data are sown below. In this instance, a to d indicate sites in the following chemical formula where respective signals are bought about.
$^1$H-NMR ($D_2O$): $\delta$ (ppm)
2.37 - 2.51 (t, 2H, a), 3.18 - 3.34 (t, 2H, c),
3.35 - 3.49 (t, 2H, b), 3.77 - 3.88 (t, 2H, d)

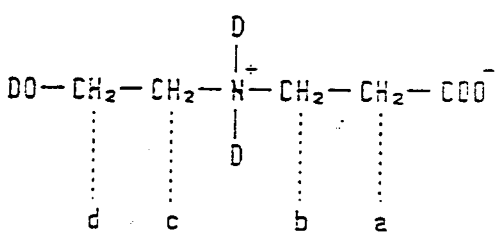

Mass spectrometry date (FAB ionization method) are shown below.
MS (m/z): 134 $(M+H)^+$, ($M = C_5H_{11}O_3N$)

EXAMPLE 6

A reactor was charged with 200.0 g (0.92 mol) of sodium 6-bromohexanoate which was subsequently dispersed in ethanol. To this was added 54.1 g (0.72 mol) of 2-(methylamino)ethanol in a dropwise manner over 1 hour at room temperature. After completion of the dropwise addition, temperature of the resulting mixture was gradually elevated to 80 ° C, and the reaction was carried out for 15 hours at the same temperature.

After completion of the reaction, the resulting reaction solution was directly subjected to purification by an ion exchange chromatography (ion exchange resin: AG501-X8 manufactured by Bio-Rad Laboratories, Inc.), followed by distillation removal of the solvent under a reduced pressure to obtain 92.5 g of 6-(N-methyl-N-hydroxyethyl)-aminohexanoic acid with a yield of 68 %.

This compound showed a purity of 99 % when analyzed by HPLC (column: Shimadzu Gel SCR101N manufactured by Shimadzu Corp.; eluent: water).

$^1$H-NMR data are sown below. In this instance, a to f indicate sites in the following chemical formula where respective signals are bought about.
$^1$H-NMR ($D_2O$): $\delta$ (ppm)
1.02 - 1.70 (m, 6H, b), 1.93 - 2.05 (t, 2H, a), 2.91 (s, 3H, d), 3.06 - 3.20 (t, 2H, c), 3.20 - 3.34 (t, 2H, e), 3.75 - 3.90 (t, 2H, f)

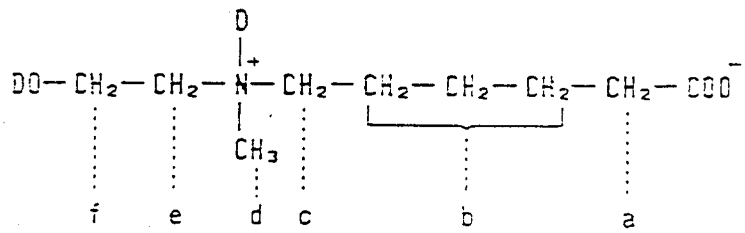

Mass spectrometry date (FAB ionization method) are shown below.
MS (m/z): 190 (M + H) , (M = $C_9H_{19}O_3N$)

EXAMPLE 7

A reactor was charged with 300 g (2.9 mol) of N,N-dimethylpropanolamine and 320 g of water, and temperature of the mixture was elevated to 55 °C. To this was subsequently added 407 g (3.5 mol) of sodium chloroacetate dissolved in 450 g of water, in a dropwise manner over 2.5 hours at the same temperature. After completion of the dropwise addition, the resulting mixture was stirred for 8 hours while keeping the reaction temperature at 55 °C. After completion of the reaction, the resulting reaction solution was directly subjected to purification by an ion exchange chromatography (ion exchange resin: AG501-X8 manufactured by Bio-Rad Laboratories, Inc.), followed by distillation removal of the solvent under reduced pressure to obtain 380 g of N,N-dimethyl-N-hydroxypropylglycine with a yield of 81 %. This compound showed a purity of 99 % when analyzed by HPLC (column: Shimadzu Gel SCR101N manufactured by Shimadzu Corp.; eluent: water).
$^1$H-NMR ($D_2O$): $\delta$ (ppm)
1.79 - 1.98 (complex multiplet, 2H, b)
3.13 (singlet, 6H, d)
3.45 - 3.62 (complex multiplet, 4H, a;c)
3.77 (singlet, 2H, e)

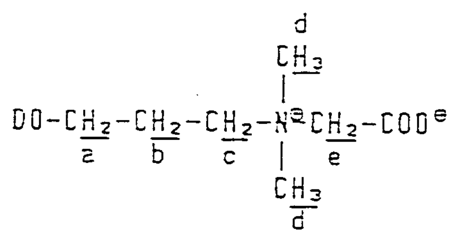

Mass spectrometry (FAB ionization method):
M/Z 162 $(M + H)^+$
(M = $C_7H_{15}O_3N$)

EXAMPLE 8

A reactor was charged with 50 g (0.36 mol) of dimethylglycine hydrochloride, 100 ml of water and 150 ml of ethanol, and the resulting mixture was adjusted to pH 9 with 48 % sodium hydroxide aqueous solution, followed by elevating the temperature to 60 °C. To the resulting mixture was subsequently added 77 g (0.40 mol) of 10-chlorodecanol dissolved in 100 ml of ethanol, in a dropwise manner over 2 hours at the same temperature. During the dropwise addition, pH level of the reaction mixture was controlled at 9 by the occasional addition of 48 % sodium hydroxide aqueous solution. After completion of the dropwise addition, the resulting mixture was stirred for 5 hours while keeping the reaction temperature at the same level. After completion of the reaction, solvent and unreacted 10-chlorodecanol were removed by distillation under a reduced pressure, and the resulting residue was dissolved in 400 ml of water and subjected to purification by an ion exchange chromatocraphy (ion exchange resin: AG501-X8 manufactured by Bio-Rad Laboratories, Inc.), followed by distillation removal of the solvent under a reduced pressure to obtain N,N-dimethyl-N-hydroxydecylglycine with a yield of 76 %. When this compound was subjected to a silica gel thin layer chromatography (developing solvent: chloroform/methanol = 1/1), it showed a single spot.

$^1H$-NMR ($D_2O$): δ (ppm)
1.21 - 1.31 (broad singlet, 12H, c)
1.60 - 1.95 (complex multiplet, 4H, b + d)
3.14 (singlet, 6H, f)
3.48 - 3.70 (complex multiplet, 4H, a + e)
3.780 (singlet, 2H, g)

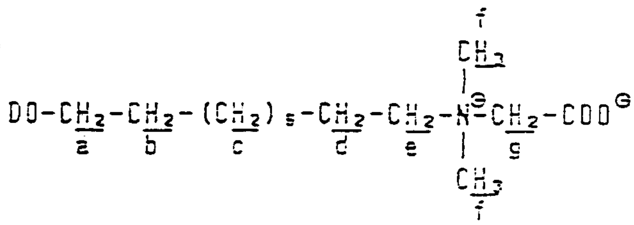

Mass spectrometry (FAB ionization method):
M/Z 260 $(M+H)^+$
(M = $C_{14}H_{29}O_3N$)

EXAMPLE 9

A reactor was charged with 217.0 g (1.0 mol) of sodium 6-bromohexanoate, 200 g of water and 400 g of ethanol. To the resulting mixture was subsequently added 62.4 g (0.7 mol) of N,N-dimethylethanolamine in a dropwise manner over 1 hour at room temperature. After completion of the dropwise addition, temperature of the resulting mixture was elevated gradually to 80 °C, followed by 8 hours of reaction at the same temperature. After completion of the reaction, the resulting reaction solution was directly subjected to purification by an ion exchange chromatography (ion exchange resin: AG501-X8 manufactured by Bio-Rad Laboratories, Inc.), followed by distillation removal of the solvent under reduced pressure to obtain 55.4 g of 1-carboxybutyl-N,N-dimethyl-N-hydroxyethyl-N-methaneaminium hydroxide inner salt with an isolation yield of 39 %. This compound showed a purity of 99.5 % when analyzed by HPLC (column: Shimadzu Gel SCR101N manufactured by Shimadzu Corp.; eluent: water; detector: RI).
$^1H$-NMR ($D_2O$): δ (ppm)

| | Proton numbers |
|---|---|
| 0.83 - 1.49 (multiplet, a) | 6H |
| 1.68 - 2.37 (triplet, b) | 2H |
| 2.72 (singlet, c) | 6H |
| 2.86 - 3.03 (broad, d) | 2H |
| 3.04 - 3.15 (triplet, e) | 2H |
| 3.52 - 3.70 (broad, f) | 2H |

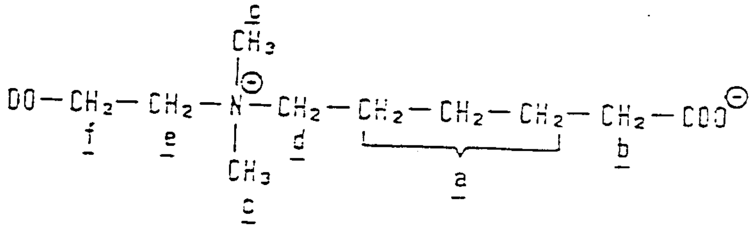

Mass spectrometry (FAB ionization method):
M/Z 274 (M + H)
M = $C_{10}H_{21}O_3N$

EXAMPLE 10

A reactor was charged with 287.0 g (1.0 mol) of sodium 11-bromoundecanoate, 200 g of water and 400 g of ethanol. To the resulting mixture was subsequently added 62.4 g (0.7 mol) of N,N-dimethylethanolamine in a dropwise manner over 1 hour at room temperature. After completion of the dropwise addition, temperature of the resulting mixture was elevated gradually to 80 °C, followed by 8 hours of reaction at the same temperature. After completion of the reaction, the resulting reaction solution was directly subjected to purification by an ion exchange chromatography (ion exchange resin: AG501-X8 manufactured by Bio-Rad Laboratories, Inc.), followed by distillation removal of the solvent under reduced pressure to obtain 44.0 g of 1-carboxydecyl-N,N-dimethyl-N-hydroxyethyl-N-methaneaminium hydroxide inner salt with an isolation yield of 23 %. This compound showed a purity of 99.7 % when analyzed by HPLC (column: Shimadzu Gel SCR101N manufactured by Shimadzu Corp.; eluent: water; detector: RI).
$^1$H-NMR ($D_2O$): δ (ppm)

| | Proton numbers |
|---|---|
| 0.70 - 1.56 (multiplet, broad, a) | 6H |
| 1.71 - 1.88 (triplet, b) | 2H |
| 2.75 (singlet, c) | 6H |
| 2.93 - 3.10 (multiplet, d) | 2H |
| 3.10 - 3.24 (multiplet, e) | 2H |
| 3.57 - 3.73 (broad, f) | 2H |

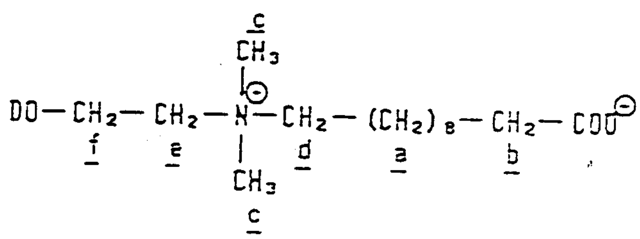

Mass spectrometry (FAB ionization method):
M/Z 274 (M + H)
M = $C_{15}H_{31}O_3N$

EXAMPLE 11

A reactor was charged with 101.9 g (1.356 mol) of 2-(methylamino)ethanol and 100 g of water. To the resulting mixture was subsequently added 385.5 (3.31 mol) of sodium chloroacetate dissolved in water, in a dropwise manner over 1 hour at room temperature. After completion of the dropwise addition, temperature of the resulting mixture was evaluated gradually to 70 °C. Thereafter, 113 g (1.356 mol) of 48% sodium hydroxide was added dropwise to the mixture, followed by 15 hours of reaction at the same temperature.

After completion of the reaction, the resulting reaction solution was dried by evaporation, and 1.5 l of methanol was added to the resulting residue to remove insoluble materials by filtration. After purification by ethanol washing, the solvent was distilled off under a reduced pressure to obtain 66 g of N-methyl-N-(1-hydroxyethyl)-N-carboxymethylglycine sodium salt.

$^1$H-NMR data are sown below. In this instance, a to d indicate sites in the following chemical formula where respective signals are bought about.
$^1$H-NMR ($D_2O$); δ (ppm) $D_2O$: 4.50 ppm
3.91 (s, 4H, a),
3.60 - 3.72 (t, 2H, b),
3.48 - 3.55 (t, 2H, c),
3.05 (s, 3H, d)

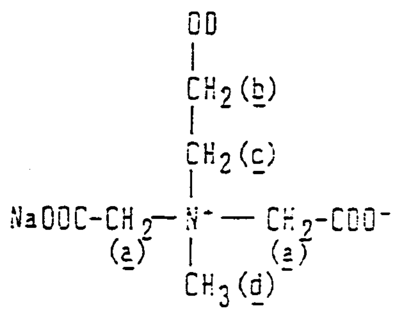

EXAMPLE 12

A reactor was charged with 50.0 g (0.48 mol) of diethanolamine and 100 g of water. To the resulting mixture was added 120.4 (1.03 mol) of sodium chloroacetate dissolved in water, in a dropwise manner over 1 hour. After completion of the dropwise addition, temperature of the resulting mixture was elevated gradually to 80 °C. Thereafter, 40 g of 48% sodium hydroxide was added dropwise to the mixture, followed by 20 hours of reaction at the same temperature.

After completion of the reaction, the resulting reaction solution was dried by evaporation, and 1 l of methanol was added to the resulting residue to remove insoluble materials by filtration. After purification by ethanol washing, the solvent was distilled off under a reduced pressure to obtain 41 g of N,N-(di-1-hydroxyethyl)-N-carboxymethylglycine sodium salt.

$^1$H-NMR data are sown below. In this instance, a to c indicate sites in the following chemical formula where respective signals are produced.

$^1$H-NMR ($D_2O$); δ (ppm) $D_2O$: 4.60 ppm
4.15 (s, 4H, a),
3.95 - 3.87 (t, 4H, b),
3.87 - 3.73 (t, 4H, c)

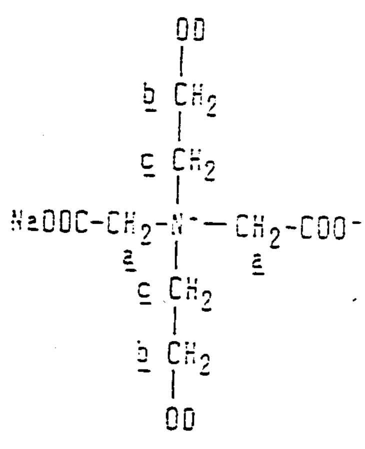

EXAMPLE 13

A 20 g portion of N-methyl-N-(1-hydroxyethyl)-N-carboxymethylglycine sodium salt obtained in Example 11 was dissolved in 100 g of water and subjected to an ion exchange chromatography (Amberlite IR-120B, manufactured by Organo Co., Ltd.) to obtain a solution of N-methyl-N-(1-hydroxyethyl)-N-carboxymethylglycine which was subsequently adjusted to pH 7.0 with triethanolamine. Thereafter, the solution was dried by evaporation to obtain 19 g of N-methyl-N-(1-hydroxyethyl)-N-carboxymethylglycine triethanolamine salt.

$^1$H-NMR data are sown below. In this instance, a to f indicate sites in the following chemical formula where respective signals are bought about.

$^1H$-NMR ($D_2O$); $\delta$ (ppm) $D_2O$: 4.70 ppm
4.05 (s, 4H, c),
3.64 - 3.92 (broad, 10H, a + d + e),
3.37 (t, 6H, b),
3.20 (s, 3H, f)

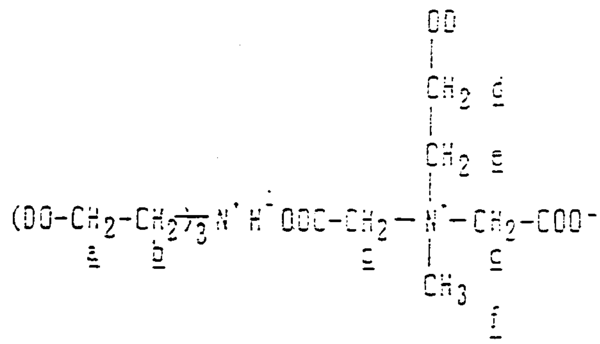

EXAMPLE 14

A solution of N-methyl-N-(1-hydroxyethyl)-N-carboxymethylglycine was obtained in the same manner as described in Example 13, adjusted to pH 7.0 with an aqueous solution of choline and then dried by evaporation to obtain N-methyl-N-(1-hydroxyethyl)-N-carboxymethylglycine choline salt.

EXAMPLE 15

Reaction of dimethylamine with sodium chloroacetate was carried out in the same manner as described in Example 11 in the presence of sodium hydroxide to obtain N,N-dimethyl-N-carboxymethylglycine sodium salt which was subsequently subjected to sodium removal by the ion exchange chromatography described in Example 13. Thereafter, the resulting solution was adjusted to pH 7.0 with triethanolamine and then dried by evaporation to obtain N,N-dimethyl-N-carboxymethylglycine triethanolamine salt.

EXAMPLE 16

(Evaluation of moisture keeping ability)

The carboxybetaines of the present invention were evaluated for their moisture keeping capacities in the following manner, with the results shown in Table 1.

Evaluation method:

Each of the test samples made into 2% by weight aqueous solution was applied to a spot on the fore arm flexor side of a panelist in an amount of 10 $\mu l$ per 1 $cm^2$ and maintained as such for 10 minutes. In this instance, the fore arm side was subjected to conditioning at 20 °C and at 40% RH in advance. Skin conductance was measured before and after this treatment using SKICON-200 (IBS Co., Ltd.) to calculate moisture keeping ability of the test sample based on the conductance ratio before and after the treatment. (N = 10)

$$\text{moisture keeping ability} = \frac{\text{after treatment conductance}}{\text{before treatment conductance}}$$

TABLE 1

| Samples | | Moisture Keeping Ability |
|---|---|---|
| 1 | N-methyl-N-(1-hydroxyethyl)-N-carboxymethylglycine sodium salt (Compound of Example 11) | 1.5 |
| 2 | N,N-di(1-hydroxyethyl)-N-carboxymethylglycine sodium salt (Compound of Example 12) | 1.4 |
| 3 | N-methyl-N-(1-hydroxyethyl)-N-carboxymethylglycine triethanolamine salt (Compound of Example 13) | 2.4 |
| 4 | N-methyl-N-(1-hydroxyethyl)-N-carboxymethylglycine choline salt (Compound of Example 14) | 2.1 |
| 5 | N,N-dimethyl-N-carboxymethylglycine triethanolamine salt (Compound of Example 15) | 2.3 |
| Comparative Example | blank (water) | 1.0 |

As is evident from the above results, the skin conductance 10 minutes after the application of the compounds of the present invention is higher than that of the blank test, thus showing excellent moisture keeping ability of the compounds of the present invention.

EXAMPLE 17

A face cleansing paste having the following composition was prepared in the usual way.

| (Composition) | (% by weight) |
|---|---|
| Sodium sesquilauryl phosphate | 25 |
| Dipotassium myristyl sulfosuccinate | 5 |
| Cocoyl diethanolamide | 2 |
| Polyethylene glycol monostearate | 4 |
| Compound of Example 11 | 5 |
| Carboxyvinyl polymer | 0.5 |
| Paraben | 0.3 |
| Perfume | 0.3 |
| Purified water | balance |

The thus obtained face cleansing paste was able to provide a neat after-wash feeling and a moist feeling without causing a tense feeling.

EXAMPLE 18

A liquid body shampoo having the following composition was prepared in the usual way.

| (Composition) | (% by weight) |
|---|---|
| Triethanolamine lauryl phosphate | 20 |
| Alkyl saccharide [$C_{12}$-O-(G)$_{2.5}$] *1 | 5 |
| Lauroylsarcosine sodium salt | 5 |
| Compound of Example 13 | 8 |
| Xanthan gum | 0.5 |
| Propylene glycol | 3 |
| Perfume | 0.7 |
| Purified water | balance |

Note: *1: $C_{12}$ is a lauryl group and G is glucose.

The thus obtained body shampoo did not cause a rough skin after washing and was able to provide a moist feeling.

EXAMPLE 19

An anti-dandruff shampoo having the following composition was prepared in the usual way.

| (Composition) | (% by weight) |
|---|---|
| Luryldimethylamine betaine acetate | 10 |
| Sodium N-lauroyl glutamate | 10 |
| Pyrocton auramine (Octopyrox, Hoechst) | 0.5 |
| Ethylene glycol distearate | 2 |
| Compound of Example 13 | 5 |
| Perfume | 0.5 |
| Water | balance |

This anti-dandruff shampoo showed no squeaky feel during hair shampooing and rinsing, and the after-wash feeling is not sticky but moist.

EXAMPLE 20

A dish washing detergent having the following composition was prepared in the usual way.

| (Composition) | (% by weight) |
|---|---|
| Sodium polyoxyethylene (4) lauryl ether sulfate | 8 |
| Polyoxyethylene (20) myristyl ether | 5 |
| Lauryldimethylamine oxide | 3 |
| Ethanol | 3 |
| Compound of Example 12 | 3 |
| Perfume | 0.1 |
| Water | balance |

This dish washing detergent did not cause dry and rough hands after its use, and was able to provide a moist feeling.

EXAMPLE 21

A hair treatment having the following composition was prepared in the usual way.

| (Composition) | (% by weight) |
|---|---|
| 2-Dodecylhexadecyltrimethylammonium chloride | 2 |
| Stearyltrimethylammonium chloride | 2 |
| Compound of Example 14 | 5 |
| Stearyl alcohol | 5 |
| Lanolin | 3 |
| Liquid paraffin | 3 |
| Polypeptide (collagen hydrolyzate) | 5 |
| Hydroxyethyl cellulose (1% aqueous solution; viscosity: 8,000 cp) | 0.5 |
| polyethylene (5) oleyl ether | 0.5 |
| Methylparaben | 0.2 |
| Perfume | 0.4 |
| Water | balance |

This hair treatment showed excellent effects in providing hair with flexibility and a moist feeling with no stickiness.

EXAMPLE 22

A face lotion having the following composition was prepared in the usual way.

| (Composition) | (% by weight) |
|---|---|
| Lactic acid | 0.03 |
| Sodium lactate | 0.84 |
| Compound of Example 15 | 5 |
| Glycerol | 2 |
| Polyoxyethylene oleyl ether (addition product of 20 EO) | 1 |
| Ethanol | 10 |
| Perfume | 0.3 |
| Water | balance |

Moisture keeping ability of this face lotion was not spoiled by sweat, and not sticky but moist feeling was obtained.

EXAMPLE 23

A bath powder having the following composition was prepared in the usual way.

| (Composition) | (% by weight) |
|---|---|
| Sodium bicarbonate | 67 |
| Dextrin | 30 |
| Compound of Example 15 | 2 |
| Perfume | 0.5 |
| Coloring matter | 0.5 |

This bath powder showed excellent moisture keeping effect and moist feeling on the skin.

EXAMPLE 24

A reactor was charged with 55.0 ml (0.5 mol) of N,N-dimethylethylenediamine, and 50.0 ml (0.5 mol) of concentrated hydrochloric acid was added thereto in a dropwise manner while cooling with an ice bath thereby making the amine compound into hydrochloride. To this were added 120.12 g (2 mol) of urea dissolved in water and a hydrochloric acid/acetic acid mixture (4 ml/4 ml), followed by 4 hours of reaction under reflux. After completion of the reaction, the reaction solution was mixed with 200 ml of 30 % sodium hydroxide aqueous solution and extracted with chloroform, and the resulting chloroform layer was dried by evaporation and dissolved again in 200 ml of water.

To the thus obtained aqueous solution was added 58.2 g (0.5 mol) of sodium chloroacetate dissolved in water, in a dropwise manner over 1 hour at room temperature. Thereafter, temperature of the resulting mixture was elevated gradually to 70 °C, followed by 15 hours of reaction at the same temperature. After completion of the reaction, the resulting reaction solution was directly subjected to purification by an ion exchange chromatography (ion exchange resin: AG-501-X8 manufactured by Bio-Rad Laboratories, Inc.), followed by distillation removal of the solvent under a reduced pressure to obtain 72.8 g of N,N-dimethyl-N-ethylureidoglycine. This compound showed a purity of 99 % when analyzed by HPLC (column: Shimadzu Gel SCR101N manufactured by Shimadzu Corp.; eluent: water; detector: RI).

$^1$H-NMR ($D_2O$); $\delta$ (ppm) $D_2O$:4.65:
3.68 (s, 2H, d),
3.55 (t, 2H, b),
3.33 (m, 2H, a),
3.07 (s, 6H, c).

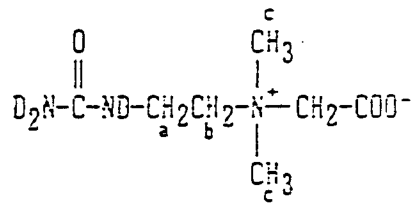

EXAMPLE 25

A reactor was charged with 76.05 g (1.0 mol) of glycolic acid, 500 ml of toluene and 132 ml (1.2 mol) of N,N-dimethylethylenediamine, followed by 20.5 hours of reaction under reflux. This reaction was carried out by trapping formed water. After completion of the reaction, the reaction solution was dried by evaporation, and the dried residue was again dissolved in 300 ml of water.

To the thus obtained aqueous solution was added 139.8 g (1.2 mol) of sodium chloroacetate dissolved in water, in a dropwise manner over 1.5 hours at room temperature. Thereafter, temperature of the resulting mixture was elevated gradually to 70 °C, followed by 12 hours of reaction at the same temperature. After completion of the reaction, the resulting reaction solution was directly subjected to purification by an ion exchange chromatography (ion exchange resin: AG-501-X8 manufactured by Bio-Rad Laboratories, Inc.), followed by distillation removal of the solvent under a reduced pressure to obtain 162 g of N,N-dimethyl-N-glycolamidoethylglycine. This compound showed a purity of 99.5 % when analyzed by HPLC (column: Shimadzu Gel SCR101N manufactured by Shimadzu Corp.; eluent: water; detector: RI).

$^1$H-NMR ($D_2O$); δ (ppm) $D_2O$:4.71:
4.05 (s, 2H, a),
3.89 (s, 2H, d),
3.55 - 3.78 (m, 4H, b),
3.18 (s, 6H, c).

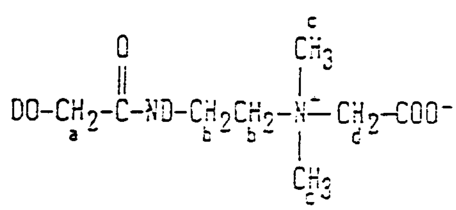

EXAMPLE 26

A reactor was charged with 129.12 g (1.0 mol) of L-2-pyrrolidone-5-carboxylicacid, 500 ml of toluene and 110 ml (1.0 mol) of N,N-dimethylethylenediamine, followed by 15.5 hours of reaction under reflux. This reaction was carried out by trapping formed water. After completion of the reaction, the reaction solution was dried by evaporation, and the dried residue was again dissolved in 400 ml of water.

To the thus obtained aqueous solution was added 128.1 g (1.1 mol) of sodium chloroacetate dissolved in water, in a dropwise manner over 2 hours at room temperature. The resulting mixture was heated gradually to 70 °C, followed by 15 hours of reaction at the same temperature. After completion of the reaction, the resulting reaction solution was directly subjected to purification by an ion exchange chromatography (ion exchange resin: AG-501-X8 manufactured by Bio-Rad Laboratories, Inc.), followed by distillation removal of the solvent under a reduced pressure to obtain 156.8 g of N,N-dimethyl-N-pyrrolidone-5-carboxyamidoethylglycine. This compound showed a purity of 99 % when analyzed by HPLC (column: Shimadzu Gel SCR101N manufactured by Shimadzu Corp.; eluent: water; detector: RI).

$^1$H-NMR ($D_2O$); δ (ppm) $D_2O$:4.55:
4.00 - 4.15 (m, 1H, b),
3.66 (s, 2H, e),
3.38 - 3.63 (m, 4H, c),
3.05 (m, 6H, d),
1.74 - 2.39 (m, 4H, a).

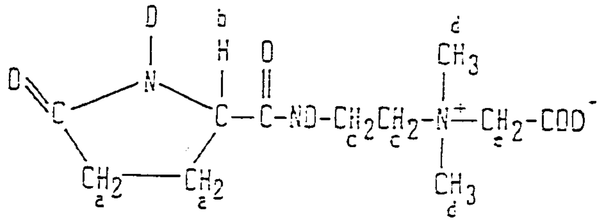

EXAMPLE 27

A hair treatment having the following composition was prepared in the usual way.

| (Composition) | (% by weight) |
| --- | --- |
| 2-Dodecylhexadecyltrimethylammonium chloride | 2 |
| Stearyltrimethylammonium chloride | 2 |
| Compound of Example 25 | 5 |
| Stearyl alcohol | 5 |
| Lanolin | 3 |
| Liquid paraffin | 3 |
| Polypeptide (collagen hydrolyzate) | 5 |
| Hydroxyethyl cellulose (1 % aqueous solution; viscosity: 8,000 cp) | 0.5 |
| Polyethylene (5) oleyl ether | 0.5 |
| Methylparaben | 0.2 |
| Perfume | 0.4 |
| Water | balance |

This hair treatment showed excellent effects in providing hair with flexibility and a moist feeling with no stickiness.

EXAMPLE 28

A face lotion having the following composition was prepared in the usual way.

| (Composition) | (% by weight) |
| --- | --- |
| Lactic acid | 0.03 |
| Sodium lactate | 0.84 |
| Compound of Example 26 | 5 |
| Glycerol | 2 |
| Polyoxyethylene oleyl ether (addition product of 20 EO) | 1 |
| Ethanol | 10 |
| Perfume | 0.3 |
| Water | balance |

Moisture keeping ability of this face lotion was not spoiled by sweat, and not sticky but moist feeling was obtained.

EXAMPLE 29

A powder bathing preparation having the following composition was prepared in the usual way.

| (Composition) | (% by weight) |
|---|---|
| Sodium bicarbonate | 67 |
| Dextrin | 30 |
| Compound of Example 24 | 2 |
| Perfume | 0.5 |
| Coloring matter | 0.5 |

This powder bathing preparation showed excellent moisture keeping effect and moist feeling on the skin.

EXAMPLE 30

A face cleansing paste having the following composition was prepared in the usual way.

| (Composition) | (% by weight) |
|---|---|
| Sodium sesquilauryl phosphate | 25 |
| Dipotassium myristyl sulfosuccinate | 5 |
| Cocoyl diethanolamide | 2 |
| Polyethylene glycol monostearate | 4 |
| Compound of Example 25 | 5 |
| Carboxyvinyl polymer | 0.5 |
| Paraben | 0.3 |
| Perfume | 0.3 |
| Purified water | balance |

The thus obtained face cleansing paste was able to provide a neat after-wash feeling and a moist feeling without causing a tense feeling.

EXAMPLE 31

A liquid body shampoo having the following composition was prepared in the usual way.

| (Composition) | (% by weight) |
|---|---|
| Triethanolamine lauryl phosphate | 20 |
| Alkyl saccharide [$C_{12}$-O-$(G)_{2.5}$] *1 | 5 |
| Lauroylsarcosine sodium salt | 5 |
| Compound of Example 25 | 8 |
| Xanthan gum | 0.5 |
| Propylene glycol | 3 |
| Perfume | 0.7 |
| Purified water | balance |

Note: *1: $C_{12}$ is a lauryl group and G is glucose.

The thus obtained body shampoo did not cause a rough skin after washing and was able to provide a moist feeling.

EXAMPLE 32

An anti-dandruff shampoo having the following composition was prepared in the usual way.

| (Composition) | (% by weight) |
|---|---|
| Lauryldimethylamine acetate betaine | 10 |
| Sodium N-lauroyl glutamate | 10 |
| Pyrocton auramine (Octopyrox, Hoechst) | 0.5 |
| Ethylene glycol distearate | 2 |
| Compound of Example 25 | 5 |
| Perfume | 0.5 |
| Water | balance |

This anti-dandruff shampoo showed no grating feel during hair shampooing and rinsing, and the after-wash feeling is not sticky but moist.

EXAMPLE 33

A dish washing detergent having the following composition was prepared in the usual way.

| (Composition) | (% by weight) |
|---|---|
| Sodium polyoxyethylene (4) lauryl ether sulfate | 8 |
| Polyoxyethylene (20) myristyl ether | 5 |
| Lauryldimethylamine oxide | 3 |
| Ethanol | 3 |
| Compound of Example 25 | 3 |
| Perfume | 0.1 |
| Water | balance |

This dish washing detergent did not cause dry and rough hands after its use, and was able to provide a moist feeling.

EXAMPLE 34

Production of N,N-dimethyl-N-(2,3-dihydroxypropyl)glycine

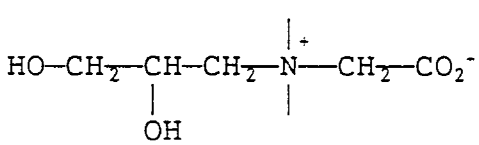

A reactor was charged with 180 g (2.0 mol) of 50 % dimethylamine aqueous solution, and 74 g (1.0 mol) of glycidol was added thereto in dropwise manner over 40 minutes. After reaction at room temperature for additional 1 hour, temperature of the reaction solution was gradually elevated to 50 °C, and water and excess dimethylamine were distilled off at the same temperature in a stream of nitrogen gas, followed by drying under a reduced pressure to obtain crude N,N-dimethyl-N-(2,3-dihydroxypropyl)amine. A reactor was charged with 61.5 g (0.5 mol) of the thus obtained amine compound and 120 g of water, and 116.5 g (1.0 mol) of sodium chloroacetate dissolved in water was added dropwise to the mixture over 40 minutes, followed by gradual evaluation of the temperature and 1 hour of reaction at 60 °C.

After completion of the reaction, the resulting reaction solution was subjected to purification by an ion exchange chromatography (ion exchange resin: AG-501-X8 manufactured by Bio-Rad Laboratories, Inc.), followed by distillation removal of the solvent under a reduced pressure to obtain 25 g of the title compound with a yield of 75 %.

EXAMPLE 35

Production of N,N-dimethyl-N-(2,6,7-trihydroxy-4-oxaheptyl)glycine

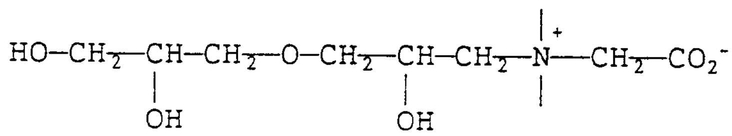

A reactor was charged with 900 g (10.2 mol) of 50 % dimethylamine aqueous solution, and 1,126 g (7.6 mol) of 2,3-dihydroxypropyl 2,3-epoxypropyl ether was added thereto in dropwise manner at room temperature over 3 hours, followed by gradual evaluation of the temperature and 4 hours of reaction at 50 ° C. After completion of the reaction, water and remaining dimethylamine were distilled off under reduced pressure to obtain 1,482 g of a crude product. To this was added 1,000 g of water to dissolve the crude product, and to the resulting solution was added 1,000 g (8.7 mol) of sodium chloroacetate dissolved in 1,000 g of water in dropwise manner over 5 hours while keeping the reaction system at 60 ° C, followed by additional 5 hours of reaction at 60 ° C. After completion of the reaction, the resulting reaction solution was directly subjected to purification by an ion exchange chromatography (ion exchange resin: AG-501-X8 manufactured by Bio-Rad Laboratories, Inc.), followed by distillation removal of the solvent under a reduced pressure to obtain 1,241 g of the title compound with a yield of 65 %.

EXAMPLE 36

Production of N,N-dimethyl-N-hydroxyethylglycine

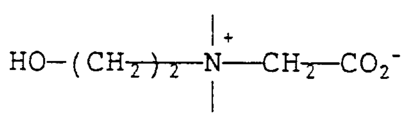

A reactor were charged with 89.1 g (1.0 mol) of N,N-dimethylethanolamine and 100 g of water, and 134.0 g (1.15 mol) of sodium chloroacetate dissolved in water was added to the resulting mixture in dropwise manner over 1 hour, followed by gradual evaluation of the temperature and 8 hours of reaction at 70 ° C. After completion of the reaction, the resulting reaction solution was subjected to purification by an ion exchange chromatography (ion exchange resin: AG-501-X8 manufactured by Bio-Rad Laboratories, Inc.), followed by distillation removal of the solvent under a reduced pressure to obtain 113.2 g of the title compound with a yield of 77 %.

EXAMPLE 37

Production of N,N-dimethyl-N-hydroxypropylglycine

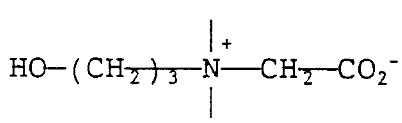

A reactor were charged with 300 g (2.9 mol) of N,N-dimethylpropanolamine and 320 g of water, and, after elevating the temperature to 55 ° C, 407 g (3.5 mol) of sodium chloroacetate dissolved in 450 g of water was added dropwise to the mixture over 2.5 hours, followed by 8 hours of stirring at 55 ° C. After completion of the reaction, the resulting reaction solution was directly subjected to purification by an ion

exchange chromatography (ion exchange resin: AG-501-X8 manufactured by Bio-Rad Laboratories, Inc.), followed by distillation removal of the solvent under a reduced pressure to obtain 380 g of the title compound with a yield of 81 %.

EXAMPLE 38

Production of N,N-dimethyl-N-hydroxyethyl-β-alanine

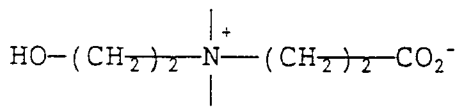

A reactor were charged with 43.3 g (0.33 mol) of sodium chloropropionate and 100 g of ethanol, and 20 g (0.22 mol) of N,N-dimethylethanolamine was added dropwise to the mixture over 1 hour, followed by gradual elevation of the temperature and 26 hours of reaction at 80 °C. After completion of the reaction, the resulting reaction solution was dried by evaporation, and the dried residue was mixed with 300 ml of methanol to remove insoluble materials by filtration. Thereafter, the solvent was distilled off, and the resulting residue was dissolved in 300 g of water and subjected to purification by an ion exchange chromatography (ion exchange resin: AG-501-X8 manufactured by Bio-Rad Laboratories, Inc.), followed by distillation removal of the solvent under a reduced pressure to obtain 12.4 g of the title compound with a yield of 34.3 %.

EXAMPLE 39

(Evaluation of moisture keeping ability and the feel)

Compounds obtained in Examples 34 to 38 and comparative compounds 1 to 4 were evaluated for their moisture keeping ability and the feel in the following manner, with the results shown in Table 2.

Evaluation of moisture keeping ability:

Each of the test samples made into 2 % aqueous solution was applied to a spot on the fore arm flexor side of a panelist in an amount of 10 µl per 1 $cm^2$ and maintained as such for 10 minutes. In this instance, the fore arm side was subjected to conditioning at 20 °C and at 40 % RH in advance. Skin conductance was measured before and after this treatment using SKICON-200 (IBS Co., Ltd.) to calculate moisture keeping ability of the test sample based on the after treatment/before treatment conductance ratio.

The above procedure was repeated 10 times, and the results were shown in the average value thereof.

Thereafter, the thus treated spot was rinsed with water, dried with a towel and then maintained as such for 10 minutes to calculate the moisture keeping ability after rinsing by measuring the skin conductance in the same manner. In this instance, the after-rinse moisture keeping ability was expressed as an after rinse/before treatment conductance ratio.

Evaluation of the feel:

Each of the test samples made into 10 % aqueous solution was applied uniformly to a spot on the fore arm flexor side of each of 10 panelists in an amount of 200 µl and maintained as such for 3 minutes. Thereafter, moist feeling and stickiness were evaluated according to the following criteria by feeling the applied spot with the palm.

(Moist feeling)

A: moist
B: slightly moist
C: not moist

(Stickiness)

A: no stickiness
B: slightly sticky
C: sticky

In this instance, the results were expressed as the average of 10 panelists.

TABLE 2

| Samples | Results of evaluation | | | |
|---|---|---|---|---|
| | Moisture keeping ability | | | |
| | before rinsing | after rinsing | Moist feel | Sticky feel |
| Product of the Invention: | | | | |
| 1 Compound of Example 34 | 1.9 | 1.7 | A | A |
| 2 Compound of Example 35 | 2.1 | 1.7 | A | A |
| 3 Compound of Example 36 | 2.7 | 2.1 | A | A |
| 4 Compound of Example 37 | 2.6 | 1.9 | A | A |
| 5 Compound of Example 38 | 2.4 | 1.8 | A | A |
| Comparative Product: | | | | |
| 1 blank (water) | 1.0 | 1.0 | C | A |
| 2 Glycerol | 3.2 | 1.3 | A | C |
| 3 Sorbitol | 1.8 | 1.0 | B | B |
| 4 N,N,N-Trimethylglycine (betaine) | 1.5 | 1.2 | B | A |

As is evident from the results shown in Table 2, each of the compounds of the present invention has excellent moisture keeping ability which is maintained even after rinsing, in addition to its excellent moist feeling and non-stickiness.

EXAMPLE 40

A face cleansing paste having the following composition was prepared in the usual way.

| (Composition) | (% by weight) |
|---|---|
| Sodium sesquilauryl phosphate | 25 |
| Dipotassium myristyl sulfosuccinate | 5 |
| Cocoyl diethanolamide | 2 |
| Polyethylene glycol monostearate | 4 |
| Compound of Example 34 | 5 |
| Carboxyvinyl polymer | 0.5 |
| Paraben | 0.3 |
| Perfume | 0.3 |
| Purified water | balance |

The thus obtained face cleansing paste was able to provide a neat after-wash feeling and a continued moist feeling.

EXAMPLE 41

A liquid body shampoo having the following composition was prepared in the usual way.

| (Composition) | (% by weight) |
|---|---|
| Triethanolamine lauryl phosphate | 20 |
| Alkyl saccharide [$C_{12}$-O-$(G)_{2.5}$] *1 | 5 |
| Lauroylsarcosine sodium salt | 5 |
| Compound of Example 36 | 8 |
| Xanthan gum | 0.5 |
| Propylene glycol | 3 |
| Perfume | 0.7 |
| Purified water | balance |

Note: *1: $C_{12}$ is a lauryl group and G is glucose.

The thus obtained body shampoo did not cause a rough skin after washing and was able to maintain a moist feeling.

EXAMPLE 42

An anti-dandruff shampoo having the following composition was prepared in the usual way.

| (Composition) | (% by weight) |
|---|---|
| Lauryldimethylamine betaine acetate | 10 |
| Sodium N-lauroyl glutamate | 10 |
| Pyrocton auramine (Octopyrox, Hoechst) | 0.5 |
| Ethylene glycol distearate | 2 |
| Compound of Example 36 | 5 |
| Perfume | 0.5 |
| Water | balance |

This anti-dandruff shampoo showed no squeaky feel during hair shampooing and rinsing, and the after-wash feeling is not sticky but moist which continued for a long time.

EXAMPLE 43

A dish washing detergent having the following composition was prepared in the usual way.

| (Composition) | (% by weight) |
|---|---|
| Sodium polyoxyethylene (4) lauryl ether sulfate | 8 |
| Polyoxyethylene (20) myristyl ether | 5 |
| Lauryldimethylamine oxide | 3 |
| Ethanol | 3 |
| Compound of Example 35 | 3 |
| Perfume | 0.1 |
| Water | balance |

This dish washing detergent did not cause dry and rough hands after its use, and a moist feeling was maintained for a long time.

EXAMPLE 44

A hair treatment having the following composition was prepared in the usual way.

| (Composition) | (% by weight) |
|---|---|
| 2-Dodecylhexadecyltrimethylammonium chloride | 2 |
| Stearyltrimethylammonium chloride | 2 |
| Compound of Example 37 | 5 |
| Stearyl alcohol | 5 |
| Lanolin | 3 |
| Liquid paraffin | 3 |
| Polypeptide (collagen hydrolyzate) | 5 |
| Hydroxyethyl cellulose (1 % aqueous solution; viscosity: 8,000 cp) | 0.5 |
| Polyethylene (5) oleyl ether | 0.5 |
| Methylparaben | 0.2 |
| Perfume | 0.4 |
| Water | balance |

This hair treatment showed excellent effects in providing hair with flexibility and a moist feeling with no stickiness, and these effects were maintained for a long time.

EXAMPLE 45

A face lotion having the following composition was prepared in the usual way.

| (Composition) | (% by weight) |
|---|---|
| Lactic acid | 0.03 |
| Sodium lactate | 0.84 |
| Compound of Example 38 | 5 |
| Glycerol | 2 |
| Polyoxyethylene oleyl ether (addition product of 20 EO) | 1 |
| Ethanol | 10 |
| Perfume | 0.3 |
| Water | balance |

Moisture keeping ability of this face lotion was not spoiled by sweat, and not sticky but moist feeling was obtained and maintained for a long time.

EXAMPLE 46

A bath powder having the following composition was prepared in the usual way.

| (Composition) | (% by weight) |
|---|---|
| Sodium bicarbonate | 67 |
| Dextrin | 30 |
| Compound of Example 38 | 2 |
| Perfume | 0.5 |
| Coloring matter | 0.5 |

This bath powder showed excellent moisture keeping effect and moist feeling on the skin for a long time.

EXAMPLE 47

Production of 1-hydroxy-2-sulfoethyl-N,N-dimethyl-N-hydroxyethyl-N-methaneaminium hydroxide inner salt

A 500 ml capacity four neck flask was charged with 18.9 g (0.21 mol) of N,N-dimethylethanolamine and 20.4 g (1.13 mol) of water, and the mixture was heated to 70 °C. To this was added a mixture of 50.1 g (0.25 mol) of sodium 3-chloro-2-hydroxy-1-propane sulfonate and 90 g (5.00 mol) of water, in a dropwise manner over about 1 hour. The resulting mixture was stirred for 7 hours at the same temperature. After cooling down to room temperature, the resulting reaction solution was directly subjected to purification by an ion exchange chromatography (ion exchange resin: AG-501-X8 manufactured by Bio-Rad Laboratories, Inc.), followed by distillation removal of the solvent under a reduced pressure to obtain 33.2 g (0.15 mol) of the title compound with a yield of 70 %. This compound showed a purity of 99.5 % when analyzed by HPLC (column: Shimadzu Gel SCR101N manufactured by Shimadzu Corp.; eluent: water; detector: RI).

Its chemical structure is shown below.

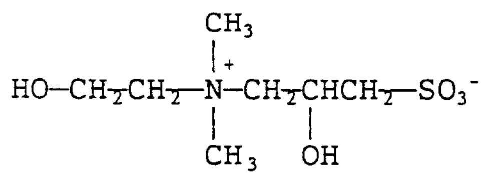

Mass spectrum data: M/Z (FAB); 228 (M + H)

A $^1$H-NMR ($D_2O$) chart is shown in Fig. 3.

EXAMPLE 48

Production of 1-hydroxy-2-sulfoethyl-N,N-dimethyl-N-(2,3-dihydroxypropyl)-N-methaneaminium hydroxide inner salt

A one liter capacity four neck flask was charged with 81 g (pure content, 0.9 mol) of 50 % aqueous solution of N,N-dimethylamine, and 32 g (0.4 mol) of glycidol was added thereto in a dropwise manner over 30 minutes.

The resulting mixture was stirred for 1 hour at room temperature and then heated to 50 °C to remove remaining dimethylamine in a stream of nitrogen. After adding 100 g of water and elevating the temperature to 60 °C, and a mixture of 164 g (0.8 mol) of sodium 3-chloro-2-hydroxy-1-propane sulfonate and 400 g of water was added to the thus resulting mixture in a dropwise manner over about 1 hour. The mixture was then stirred for 5 hours at the same temperature. After cooling down to room temperature, the resulting reaction solution was directly subjected to purification by an ion exchange chromatography (ion exchange resin: AG-501-X8 manufactured by Bio-Rad Laboratories, Inc.), followed by distillation removal of the solvent under reduced pressure to obtain 67 g (0.26 mol) of the title compound with a yield of 65 %.

Its chemical structure is shown below.

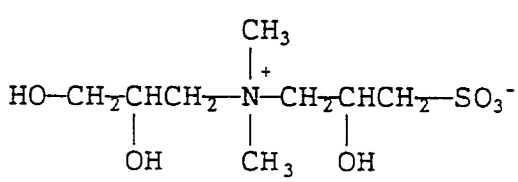

Mass spectrum data: M/Z (FAB); 257 (M + H)

A $^1$H-NMR ($D_2O$) chart is shown in Fig. 4.

EXAMPLE 49

An anti-dandruff shampoo having the following composition was prepared in the usual way.

| (Composition) | (% by weight) |
|---|---|
| Lauryldimethylamine acetate betaine | 10 |
| Sodium N-lauroyl glutamate | 10 |
| Pyrocton auramine (Octopyrox, Hoechst) | 0.5 |
| Ethylene glycol distearate | 2 |
| Compound of Example 47 | 5 |
| Perfume | 0.5 |
| Water | balance |

This anti-dandruff shampoo showed no grating feel during hair shampooing and rinsing, and the after-wash feeling is not sticky but moist.

EXAMPLE 50

A hair treatment having the following composition was prepared in the usual way.

| (Composition) | (% by weight) |
|---|---|
| 2-Dodecylhexadecyltrimethylammonium chloride | 2 |
| Stearyltrimethylammonium chloride | 2 |
| Compound of Example 47 | 5 |
| Stearyl alcohol | 5 |
| Lanolin | 3 |
| Liquid paraffin | 3 |
| Polypeptide (collagen hydrolyzate) | 5 |
| Hydroxyethyl cellulose (1 % aqueous solution; viscosity: 8,000 cp) | 0.5 |
| Polyethylene (5) oleyl ether | 0.5 |
| Methylparaben | 0.2 |
| Perfume | 0.4 |
| Water | balance |

This hair treatment showed excellent effects in providing hair with flexibility and a moist feeling with no stickiness.

EXAMPLE 51

A face lotion having the following composition was prepared in the usual way.

| (Composition) | (% by weight) |
|---|---|
| Lactic acid | 0.03 |
| Sodium lactate | 0.84 |
| Compound of Example 47 | 5 |
| Glycerol | 2 |
| Polyoxyethylene oleyl ether (addition product of 20 EO) | 1 |
| Ethanol | 10 |
| Perfume | 0.3 |
| Water | balance |

Moisture keeping ability of this face lotion was not spoiled by sweat, and not sticky but moist feeling was obtained.

EXAMPLE 52

A powder bathing preparation having the following composition was prepared in the usual way.

| (Composition) | (% by weight) |
|---|---|
| Sodium bicarbonate | 67 |
| Dextrin | 30 |
| Compound of Example 47 | 2 |
| Perfume | 0.5 |
| Coloring matter | 0.5 |

This powder bathing preparation showed excellent moisture keeping effect and moist feeling on the skin.

EXAMPLE 53

A face cleansing paste having the following composition was prepared in the usual way.

| (Composition) | (% by weight) |
|---|---|
| Sodium sesquilauryl phosphate | 25 |
| Dipotassium myristyl sulfosuccinate | 5 |
| Cocoyl diethanolamide | 2 |
| Polyethylene glycol monostearate | 4 |
| Compound of Example 47 | 5 |
| Carboxyvinyl polymer | 0.5 |
| Paraben | 0.3 |
| Perfume | 0.3 |
| Purified water | balance |

The thus obtained face cleansing paste was able to provide a neat after-wash feeling and a moist feeling without causing a tense feeling.

EXAMPLE 54

A liquid body shampoo having the following composition was prepared in the usual way.

| (Composition) | (% by weight) |
|---|---|
| Triethanolamine lauryl phosphate | 20 |
| Alkyl saccharide [$C_{12}$-O-$(G)_{2.5}$] *1 | 5 |
| Lauroylsarcosine sodium salt | 5 |
| Compound of Example 47 | 8 |
| Xanthan gum | 0.5 |
| Propylene glycol | 3 |
| Perfume | 0.7 |
| Purified water | balance |

Note: *1: $C_{12}$ is a lauryl group and G is glucose.

The thus obtained body shampoo did not cause a rough skin after washing and was able to provide a moist feeling.

EXAMPLE 55

A dish washing detergent having the following composition was prepared in the usual way.

| (Composition) | (% by weight) |
|---|---|
| Sodium polyoxyethylene (4) lauryl ether sulfate | 8 |
| Polyoxyethylene (20) myristyl ether | 5 |
| Lauryldimethylamine oxide | 3 |
| Ethanol | 3 |
| Compound of Example 48 | 3 |
| Perfume | 0.1 |
| Water | balance |

This dish washing detergent did not cause dry and rough hands after its use, and was able to provide a moist feeling.

EXAMPLE 56

A cosmetic pack of the following composition was prepared in the usual way.

| (Composition) | (% by weight) |
|---|---|
| Polyvinyl alcohol*1 | 12 |
| Polyethyleneglycol 4000 | 2 |
| Polyoxyethylene methyl glucoside 20 EO adduct*2 | 3 |
| N,N-dimethyl-N-(2-hydroxyethyl)glycine | 5 |
| Squalane | 3 |
| Ethanol | 7.7 |
| Perfume | 0.5 |
| Preservative | Appropriate amount |
| Sorbitan monostearate*3 | 0.5 |
| Polyoxyethylene sorbitan monostearate 20 EO adduct*4 | 0.2 |
| Purified water | balance |

Notes;
*1: Gosenol EG-30, trade name, manufactured by Nippon Gosei Kagaku Kogyo Co., Ltd.
*2: Glucam E-20, trade name, manufactured by Amacoal Corp.
*3: Leodol SPS10, trade name, manufactured by Kao Corp.
*4: Leodol TWS120, trade name, manufactured by Kao Corp.

This cosmetic pack showed an excellent moisturizing effect to the skin which was hard to be removed by prespiration and a good moist feel remained to the skin.

EXAMPLE 57

A cosmetic lotion of the following composition was prepared in the usual way.

| (Composition) | (% by weight) |
|---|---|
| Lactic acid | 0.03 |
| Sodium lactate | 0.84 |
| N,N-bis(2-hydroxyethyl)-N-methylglycine | 5 |
| Glycerol | 2 |
| Polyoxyethylene oleyl ether 20 EO adduct | 1 |
| Ethanol | 10 |
| Perfume | 0.3 |
| Water | balance |
| Total | 100 |

This cosmetic lotion showed excellent moisturizing effect to the skin which was hard to to be removed by perspiration and a good moist feel remained to the skin.

While the invention has been described in detail and with reference to specific examples thereof, it will be apparent to one skilled in the art that various changes and modifications can be made therein without departing from the spirit and scope thereof.

**Claims**

**1.** A carboxybetaine represented by the following formula (1):

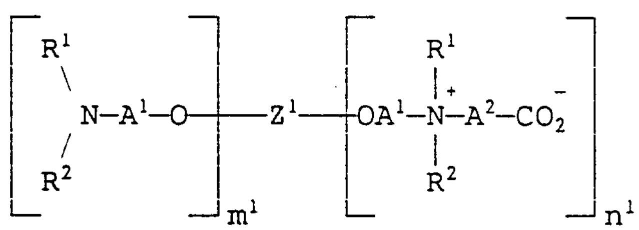

(1)

wherein $Z^1$ represents a residue remaining after removal of $m^1 + n^1$ hydroxyl groups from glycerol or a condensate thereof; $R^1$ and $R^2$ are the same or different and each represents hydrogen atom or methyl group; $A^1$ and $A^2$ are the same or different and each represents a straight- or branched-chain alkylene group having 1 to 6 carbon atoms, which may contain a hydroxyl group; and $m^1$ represents an integer of 0 or more and $n^1$ represents an integer of 1 or more, provided that $m^1 + n^1$ is equivalent to the valency of $Z^1$.

**2.** A carboxybetaine represented by the following formula (6):

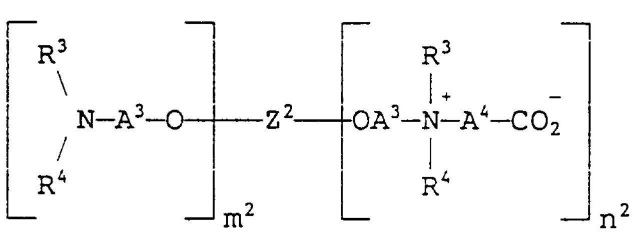

(6)

wherein $Z^2$ represents a residue remaining after removal of $m^2 + n^2$ hydroxyl groups from glycerol or a condensate thereof; $A^3$ and $A^4$ are the same or different and each represents a straight- or branched-chain alkylene group having 1 to 6 carbon atoms which may contain a hydroxyl group; $R^3$ represents a

straight- or branched-chain alkyl or alkenyl group having 1 to 24 carbon atoms which may contain a hydroxyl group; $R^4$ represents a straight- or branched-chain alkyl or alkenyl group having 2 to 24 carbon atoms which may contain a hydroxyl group; and $m^2$ represents an integer of 0 or more and $n^2$ represents an integer of 1 or more, provided that $m^2 + n^2$ is equivalent to the valency of $Z^2$.

**3.** A carboxybetaine represented by the following formula (11):

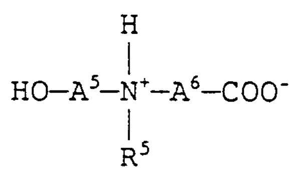

(11)

wherein $A^5$ represents a straight- or branched-chain alkylene group having 2 to 36 carbon atoms; $A^6$ represents a straight- or branched-chain alkylene group having 2 to 36 carbon atoms which may contain a hydroxyl group; and $R^5$ represents hydrogen atom or a straight- or branched-chain alkyl group having 1 to 36 carbon atoms.

**4.** A carboxybetaine represented by the following formula (14):

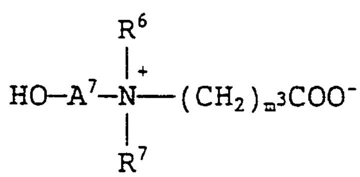

(14)

wherein $A^7$ represents a straight- or branched-chain alkylene group having 3 to 36 carbon atoms; $R^6$ and $R^7$ are the same or different and each represents a hydrogen atom, a straight- or branched-chain alkyl group having 1 to 36 carbon atoms or an alkenyl group having 2 to 36 carbon atoms; and $m^3$ represents an integer of 1 or 2.

**5.** A cosmetic composition comprising:
(a) a carboxybetaine represented by the following formula (14' ):

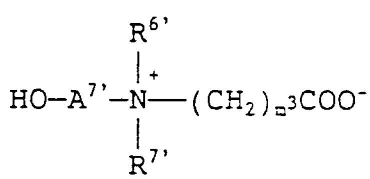

(14')

wherein $A^{7'}$ represents a straight- or branched-chain alkylene group having 2 to 36 carbon atoms; $R^{6'}$ and $R^{7'}$ are the same or different and each represents a hydrogen atom, a straight- or branched-chain alkyl group having 1 to 36 carbon atoms or an alkenyl group having 2 to 36 carbon atoms each of which may be substituted with a hydroxyl group; and $m^3$ represents an integer of 1 or 2; and
(b) a cosmetic base.

**6.** Use of a carboxybetaine represented by the following formula (14') for the production of a moisture keeping agent:

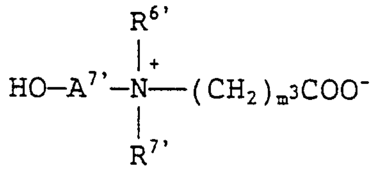

(14')

wherein $A^{7'}$ represents a straight- or branched-chain alkylene group having 2 to 36 carbon atoms; $R^{6'}$ and $R^{7'}$ are the same or different and each represents hydrogen atom, a straight- or branched-chain alkyl group having 1 to 36 carbon atoms or an alkenyl group having 2 to 36 carbon atoms each of which may be substituted with a hydroxyl group; and $m^3$ represents an integer of 1 or 2.

**7.** A detergent composition comprising:
(a) from 0.5 to 50 % by weight of a carboxybetaine represented by the following formula (14'):

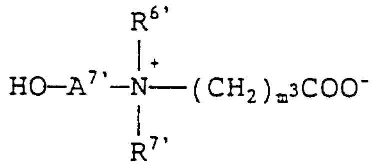

(14')

wherein $A^{7'}$ represents a straight- or branched-chain alkylene group having 2 to 36 carbon atoms; $R^{6'}$ and $R^{7'}$ are the same or different and each represents a hydrogen atom, a straight- or branched-chain alkyl group having 1 to 36 carbon atoms or an alkenyl group having 2 to 36 carbon atoms each of which may be substituted with a hydroxyl group; and $m^3$ represents an integer of 1 or 2; and
(c) from 2 to 60 % by weight of a surfactant.

**8.** A carboxybetaine represented by the following formula (19):

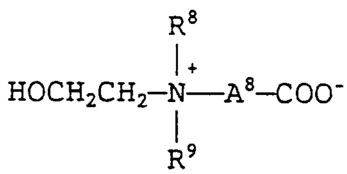

(19)

wherein $A^8$ represents a straight- or branched-chain alkylene group having 3 to 36 carbon atoms which may be substituted with a hydroxyl group; and $R^8$ and $R^9$ are the same or different and each represents a straight- or branched-chain alkyl group having 1 to 36 carbon atoms or an alkenyl group having 2 to 36 carbon atoms.

**9.** A carboxybetaine represented by the following formula (22):

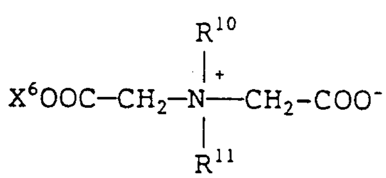

(22)

wherein $R^{10}$ and $R^{11}$ are the same or different and each represents a straight- or branched-chain alkyl group having 1 to 5 carbon atoms which may contain a hydroxy group, provided that at least one of the alkyl groups represented by $R^{10}$ and $R^{11}$ contains a hydroxy group; and $X^6$ represents hydrogen atom or a cation.

**10.** A carboxybetaine represented by the following formula (25):

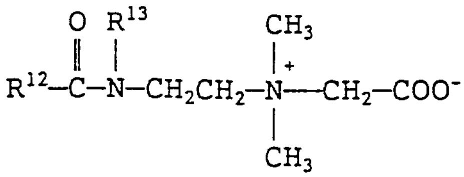

(25)

wherein $R^{12}$ represents an alkyl group having 1 to 6 carbon atoms which may be substituted with a hydroxyl group,

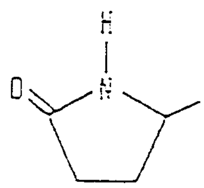

or

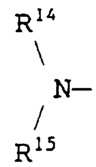

wherein $R^{14}$ and $R^{15}$ are the same or different and each represents hydrogen atom or an alkyl groups having 1 to 6 carbon atoms which may be substituted with a hydroxyl group; and $R^{13}$ represents hydrogen atom or an alkyl group having 1 to 6 carbon atoms which may be substituted with a hydroxyl group.

**11.** A carboxybetaine represented by the following formula (32):

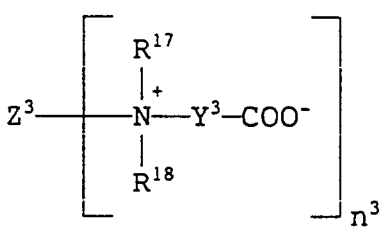

(32)

wherein $Z^3$ represents a residue remaining after removal of $n^3$ hydroxyl groups from glycerol or a condensate thereof or a group represented by HO—$A^9$— where $A^9$ represents a straight- or branched-chain alkylene group having 2 to 5 carbon atoms; $R^{17}$ and $R^{18}$ are the same or different and each represents a straight- or branched-chain alkyl group having 1 to 5 carbon atoms; $Y^3$ represents a straight- or branched-chain alkylene group which may contain a hydroxyl group, and $n^3$ represents an integer of at least 1 but not exceeding the number of hydroxyl groups in glycerol or a condensate thereof and is 1 when $Z^3$ is HO—$A^9$—.

**12.** A sulfobetaine represented by the following formula (35):

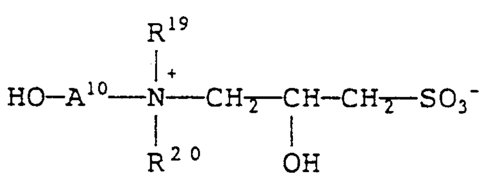

(35)

wherein $A^{10}$ represents a straight- or branched-chain alkylene group having 2 to 12 carbon atoms which may be substituted with a hydroxyl group; $R^{19}$ and $R^{20}$ are the same or different and each represents a

straight- or branched-chain alkyl group having 1 to 12 carbon atoms or a straight- or branched-chain alkenyl group having 2 to 12 carbon atoms each of which may be substituted with a hydroxyl group.

Fig. 1

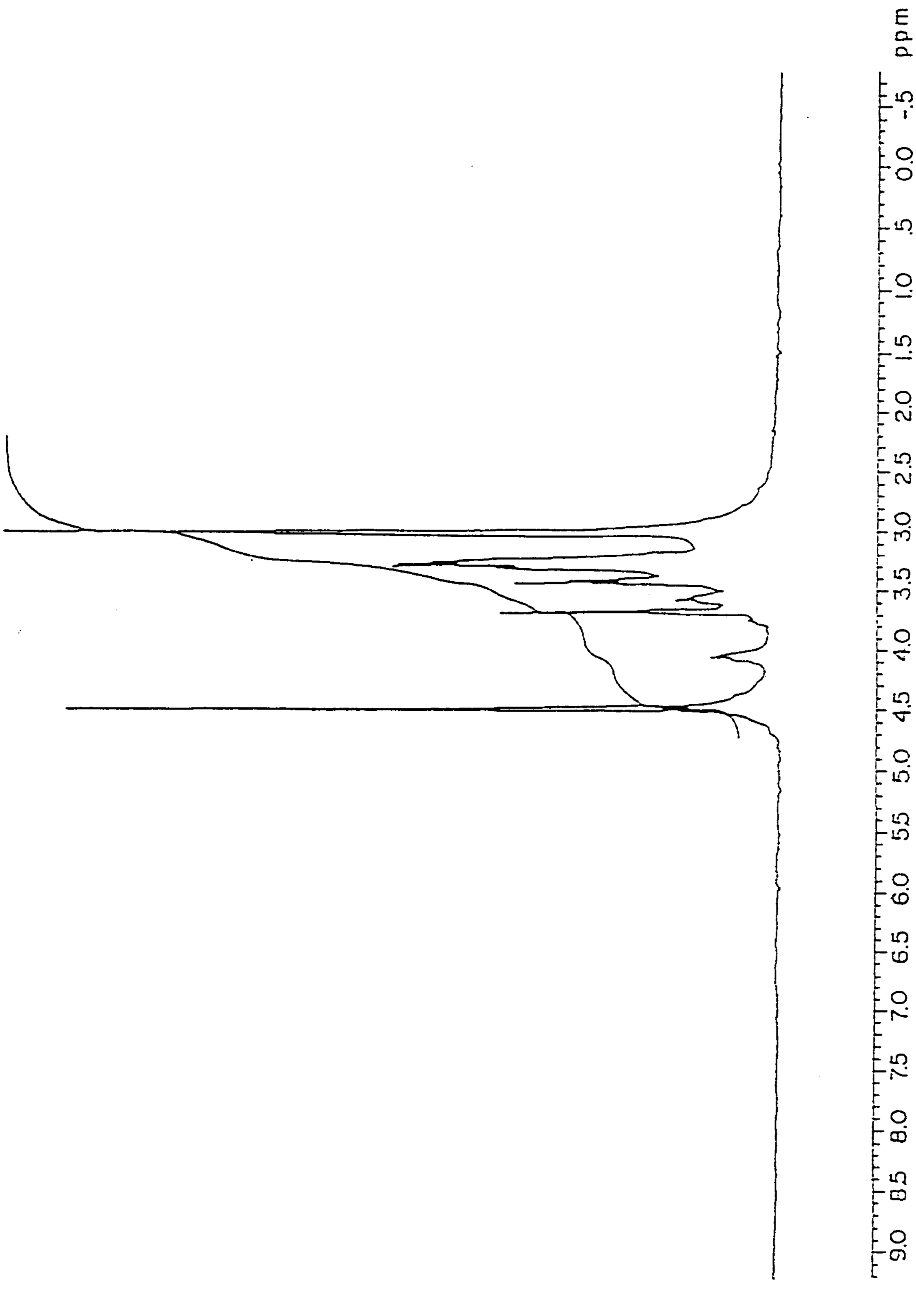

Fig. 2

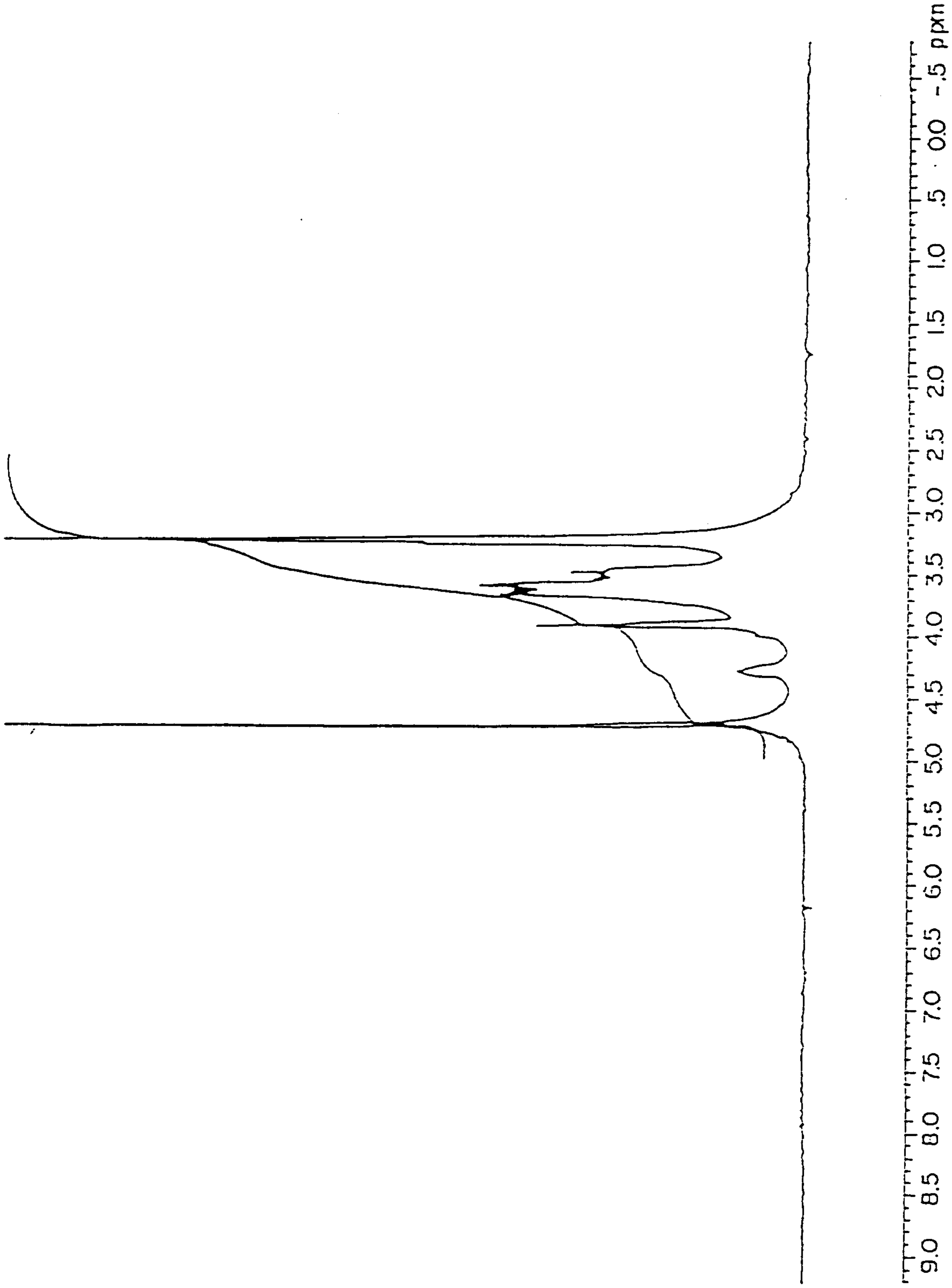

Fig. 3

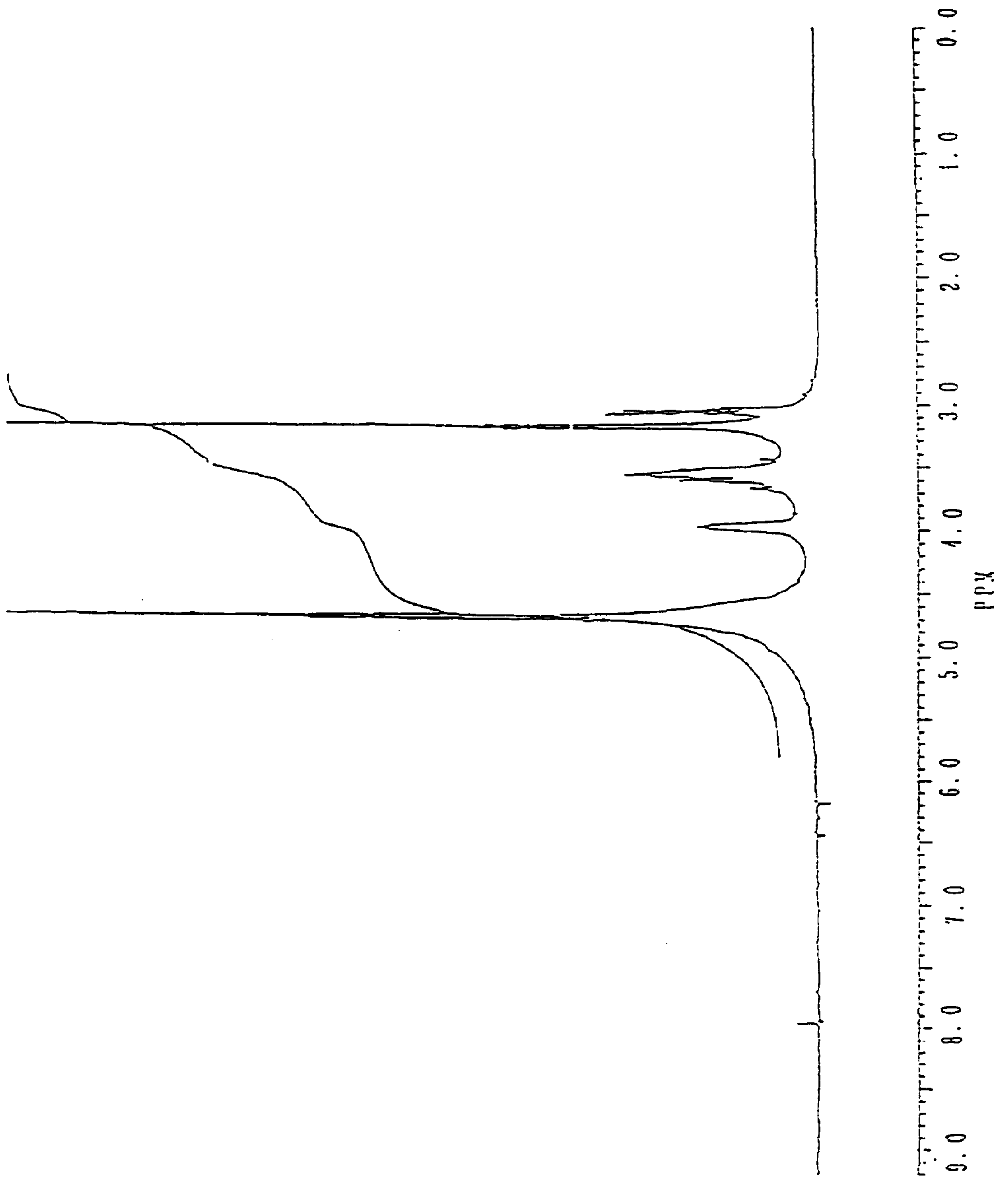

Fig. 4

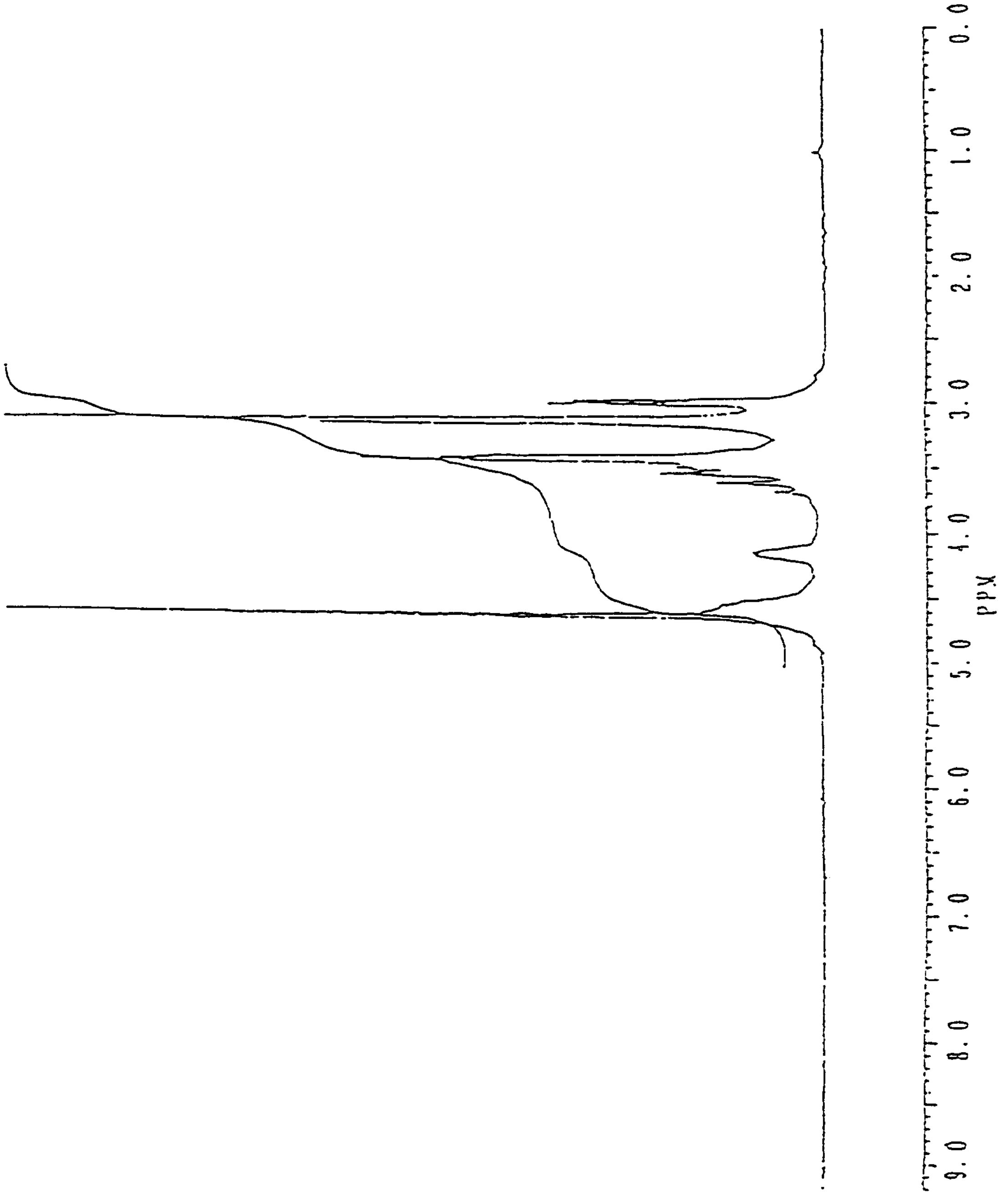

European Patent Office

# EUROPEAN SEARCH REPORT

Application Number
EP 93 11 6968

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | JOURNAL OF THE AMERICAN CHEMICAL SOCIETY., vol.73, no.7, 6 July 1951, GASTON, PA US pages 3168 - 3171 T.L. GRESHAM ET AL. 'BETA-PROPIOLACTONE.' * page 3171 * --- | 1 | C07C229/06 C07C229/08 C07C229/12 A61K7/50 A61K7/48 A61K7/06 |
| X | EP-A-0 563 747 (HOECHST AG.) * the whole document * --- | 1,5,8 | C11D1/90 C11D1/92 C07C309/14 |
| X | EP-A-0 260 205 (LESIEUR-COTELLE) * abstract * --- | 1,5,7 | C07C275/14 C07D207/26 |
| X | DE-A-20 24 962 (KAO SOAP CO. LTD) * claim 1 * | 1,5,7 | |
| A | * claim 1 * --- | 9 | |
| X | DE-A-23 64 440 (HENKEL & CIE GMBH) * claim 1 * --- | 1 | |
| A | EP-A-0 396 871 (HÜLS AG.) * page 1 - page 3 * --- | 1 | TECHNICAL FIELDS SEARCHED (Int.Cl.6) |
| A | EP-A-0 233 842 (CIBA-GEIGY AG) * the whole document * --- | 10 | C07C A61K C11D C07D |
| A | EP-A-0 077 674 (UNILEVER NV) * claim 1 * --- | 10 | |
| X | EP-A-0 439 186 (KAO CORPORATION) * page 6 - page 7 * --- | 12 | |
| X | EP-A-0 213 054 (SHEREX CHEMICAL COMPANY, INC.) * the whole document * --- | 12 | |
| | -/-- | | |

The present search report has been drawn up for all claims

| Place of search | Date of completion of the search | Examiner |
|---|---|---|
| BERLIN | 2 August 1994 | Rufet, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 93 11 6968

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DATABASE WPI<br>Week 7845,<br>Derwent Publications Ltd., London, GB;<br>AN 78-81119A<br>& JP-A-53 113 786 (KAO SOAP KK) 4 October 1978<br>* abstract *<br>--- | 12 | |
| A | WO-A-93 15259 (COMMONWEALTH SCIENTIFIC AND INDUSTRIAL RESEARCH ORGANISATION)<br>* abstract *<br>----- | 12 | |
| | | | TECHNICAL FIELDS SEARCHED (Int.Cl.6) |

The present search report has been drawn up for all claims

| Place of search | Date of completion of the search | Examiner |
|---|---|---|
| BERLIN | 2 August 1994 | Rufet, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

EP 93116968

## CLAIMS INCURRING FEES

The present European patent application comprised at the time of filing more than ten claims.

- [ ] All claims fees have been paid within the prescribed time limit. The present European search report has been drawn up for all claims.
- [ ] Only part of the claims fees have been paid within the prescribed time limit. The present European search report has been drawn up for the first ten claims and for those claims for which claims fees have been paid.

  namely claims:
- [ ] No claims fees have been paid within the prescribed time limit. The present European search report has been drawn up for the first ten claims.

## [X] LACK OF UNITY OF INVENTION

The Search Division considers that the present European patent application does not comply with the requirement of unity of invention and relates to several inventions or groups of inventions,

namely:

See Annex.

- [X] All further search fees have been paid within the fixed time limit. The present European search report has been drawn up for all claims.
- [ ] Only part of the further search fees have been paid within the fixed time limit. The present European search report has been drawn up for those parts of the European patent application which relate to the inventions in respect of which search fees have been paid.

  namely claims:
- [ ] None of the further search fees has been paid within the fixed time limit. The present European search report has been drawn up for those parts of the European patent application which relate to the invention first mentioned in the claims.

  namely claims: